Europäisches Patentamt

(19) European Patent Office

Office européen des brevets

(11) Publication number: **0 454 485 A2**

# (12) EUROPEAN PATENT APPLICATION

(21) Application number: **91303799.0**

(22) Date of filing: **26.04.91**

(51) Int. Cl.⁵: **C12N 15/32, C12N 15/31,
A01N 63/02, // C12N15/63 ,
(C12N15/31, C12R1:01)**

(30) Priority: **27.04.90 GB 9009571**

(43) Date of publication of application:
**30.10.91 Bulletin 91/44**

(84) Designated Contracting States:
**BE CH DE FR GB LI NL**

(71) Applicant: **NATIONAL UNIVERSITY OF
SINGAPORE
10 Kent Ridge Crescent
Singapore 0511 (SG)**

(72) Inventor: **Thanabalu, Thirumaran S/O, Inst. of
M. and Cell B.
National U. of Singapore, 10 Kent Ridge
Crescent
Singapore 0511 (SG)**

Inventor: **Oei, Coreen Quee-Hong, Inst. of M.
and Cell B.
National U. of Singapore, 10 Kent Ridge
Crescent
Singapore 0511 (SG)**
Inventor: **Berry, Colin , Inst. of M. and Cell B.
National U. of Singapore, 10 Kent Ridge
Crescent
Singapore 0511 (SG)**
Inventor: **Hindley, John, Inst. of M. and Cell B.
National U. of Singapore, 10 Kent Ridge
Crescent
Singapore 0511 (SG)**
Inventor: **Brenner, Sydney
17B St. Edward's Passage
Cambridge CB2 3PJ (GB)**

(74) Representative: **Woods, Geoffrey Corlett et al
J.A. KEMP & CO. 14 South Square Gray's Inn
London WC1R 5LX (GB)**

(54) Expression of insecticidal proteins.

(57) <u>Caulobacter</u> which express an insecticidal toxin, such as <u>Bacillus</u> <u>thuringiensis</u> toxin or <u>B.sphaericus</u> toxin, are useful for controlling insects such as mosquitos which have an aquatic phase in their life cycle.

EP 0 454 485 A2

Figure 1

(a)   pJC2297-2

(b)   pCC2297-M

(c)   pCK2297-M

The present invention relates to the expression of insecticidal proteins and to their use to kill insect larvae.

Several strains of Bacillus species, notably B. thuringiensis (B.t.) and B. sphaericus (B.s.), accumulate potent toxins against a variety of insect larvae. These toxins are in some cases concentrated in a proteinaceous 'crystal' within the bacterial spore. The filter feeding larvae of several insect species ingest these spores which are then broken down in the larval gut to release the toxins. These interact with receptor cells lining the gut. This causes swelling and detachment of the target cells followed by cessation of feeding and death of the larvae.

The toxic proteins found in strains of B.t., and more recently, those found in strains of B.s. have generated a great deal of interest in respect of their potential for use as insecticides. Various formulations of both B.t. and B.s. are in current commercial use in different parts of the world.

While the types of formulation vary with different manufacturers, they all basically consist of powders or concentrates from large scale fermentation cultures of the particular B.t. or B.s. species. Various wetting agents and preservatives may be added to improve dispersal and storage but in general the insecticide is contained within, or partially purified from, the cells or spores of the particular Bacillus species.

Various B.t. and B.s. formulations can be highly effective in the local control of certain insect populations. However, all such preparations suffer from two serious disadvantages. The first is a lack of persistency of the active agent in the local environment due to biodegradation and inability of the agent to recycle in the aqueous environment. The other problem is the rapid dilution of the toxin in the surface layer of the pond (or body of water) which is the main feeding zone of the aquatic larvae. Run-off, and especially sinking of the dense-toxin containing spore to the bottom, results in rapid depletion of the particulate toxin from the feeding zone.

We have devised a radically different way of presenting the toxin to an aqueous environment. This has involved isolating and cloning the genes for the toxins followed by in vitro genetic manipulation. These modified toxin genes are then introduced into another aquatic organism, Caulobacter, in such a way that they are maintained and expressed in the new host. These genetically transformed Caulobacter colonize and multiply in the surface layer of water where they persist and compete as a food source for insect larvae. These transformed Caulobacter are an effective killing agent for various types of mosquito larvae.

Accordingly, the present invention provides Caulobacter which express an insecticidal protein. Typically the protein is an insecticidal toxin of a strain of Bacillus or a modified insecticidally-active form of the toxin. A modified form of the toxin may be one which comprises one or more amino acid substitutions, deletions or insertions and/or an extension at either or each end. There may be a degree of homology of at least 80%, for example at least 90%, between the modified insecticidally active form of the toxin and the natural form of the toxin.

Preferably the insecticidal toxin is a B.t. toxin or B.s. toxin. The B.t. toxin may be a 130kDa toxin from B.t. var. israelensis (Goldberg and Margalit, 1977, Mosq. News 37, 353-358). The B.s. toxin may be a pair of proteins of 51.4kDa and 41.9kDa which are associated with the parasporal crystal, for example as in the case of B.s. 2297 (Wiekreniesinghe and Mendis, 1980, Mosq. News 40, 387-389. The B.s. toxin may be a 100kDa protein which is the mosquitocidal toxin from B.s. SSII-1 (Singer, 1973, Nature 244, 110-111).

The recombinant Caulobacter of the invention is typically a strain of Caulobacter in which the gene(s) encoding the insecticidal protein(s) is (are) introduced under the control of either their natural promoter or a heterologous promoter. "Heterologous" means that the promoter is not the promoter which naturally directs the expression of the protein. Where the insecticidal protein is B.s. toxin or B.t. toxin, the promoter is not therefore the promoter which naturally directs the expression of the toxin in B. sphaericus or B. thuringiensis respectively. A suitable heterologous promoter is the tac promoter. A promoter which is inducible or which directs constitutive expression may be employed.

The Caulobacter should express the insecticidal protein in an amount which is not lethal to the Caulobacter. For this purpose, it may be necessary to provide a repressor gene such as the lacI$^Q$ gene in the case of the tac promoter.

Caulobacter which express an insecticidal protein are prepared by transforming a strain of Caulobacter with a gene encoding the insecticidal protein such that the protein is expressed by the transformed Caulobacter strain. Any appropriate strain of Caulobacter may be transformed, for example C. crescentus. Preferably a strain native to the environment in which it is wished to use the transformed Caulobacter to control insect larvae is employed.

Transformation may be achieved using an expression vector encoding the insecticidal protein. A plasmid vector is generally provided for this purpose which contains the insecticidal protein gene. The vector is capable of expressing the protein when provided in Caulobacter. The gene is provided with appropriate transcriptional and translational control elements, in particular a promoter operably linked to the protein coding sequence, a transcriptional termination site and translational start and stop codons. The vector also comprises an origin of replication and, preferably, a marker gene which enables transformed Caulobacter to be selected. The marker gene may be an antibiotic resistance gene.

Alternatively, transformation may be achieved by introducing into Caulobacter cells a DNA fragment which contains or comprises the insecticidal protein gene(s), together with appropriate transcriptional and translational control elements, flanked by DNA sequences which permit homologous recombination to occur, thus enabling integration into the chromosomal DNA of the Caulobacter. The flanking sequences may be native Caulobacter DNA. Homologous recombination can then occur, enabling the DNA fragment comprising the insecticidal protein gene to be integrated into the chromosomal DNA of the Caulobacter. The DNA fragment may be introduced into the Caulobacter cells in the form of a plasmid or a linear fragment.

The use of recombinant Caulobacter species expressing insecticidal toxins, preferably Bacillus toxin genes, has considerable potential for controlling insect vectors. Caulobacter are an important, and often the predominant, species found in waters which are also the breeding grounds for mosquito and other insect larvae. Genetically modified Caulobacter can therefore be used as a means for delivering bioinsecticidal toxins into the feeding zone of insect larvae.

The invention therefore further provides a method of controlling insects which have an aquatic phase in their life cycle, which method comprises applying to the surface of water at a selected locus Caulobacter which express an insecticidal protein. The locus is typically a larval feeding zone. It may be a wetland. An insecticidal composition is also provided which comprises the recombinant Caulobacter and a suitable carrier or diluent.

The Caulobacter may be applied to the water surface as a suspension or slurry by means of droppers, drip-feed reservoirs and spray equipment or in time-release formulations. The Caulobacter are viable and reproduce on the water surface. Insecticidal protein is produced within the Caulobacter. Insect larvae ingest the Caulobacter and the insecticidal protein then kills the larvae. Insect larvae which can be controlled in this way include Culex, Anopheles, Mansonia, Psorophora, Aedes and, where the insecticidal protein is B.t. toxin, blackfly.

The amount of transformed Caulobacter added to surface water or wetland depends on a number of factors. The Caulobacter applied act partly as an inoculum and increase in number for a period of time. The extent of the increase depends on, for example, the stability of the transformed gene, the nutritional status of the environment and competition from resident flora. The amount of inoculum and frequency of application may be determined by routine experimentation, taking these factors into account. Typically, though, 5 to 25 ml of a slurry of the transformed Caulobacter may be applied per m².

The invention therefore brings together three disparate features and observations and uses these as a basis for a new approach to the problem of insect control. These are:

(i) The ubiquitous distribution of Caulobacter in the biosphere and their physiological characteristics, namely their concentration in the surface layers of water and their unusual property of attaching themselves, via a stalk-like adhesive prostheca, to almost any solid particulate support.

(ii) The feeding characteristics of insect larvae. As filter feeders solid matter is preferentially ingested (bacteria, yeast, small particulate matter) and the main feeding zone is at the water surface. This precisely matches the preferred distribution of Caulobacter and it is likely that, in the wild, Caulobacter form part of the natural diet of insect larvae.

(iii) The protein toxins synthesized by B.t. and B.s. are very potent and are highly specific for different insect larvae. B.t. and B.s. are often part of the normal flora found in these aqueous environments and the toxins are part of the natural ecosystem. As such their properties are matched to the environment in which they occur and no 'foreign' gene pool is being introduced.

The invention has the additional advantage that it can be applied to kill specific insect larvae in a targeted way. This is because Bacillus toxins are themselves highly specific and affect only a narrow range of insect larvae. The B.s. toxins are lethal to the larvae of Culex, Anopheles, Psorophora and Mansonia with lower toxicity to Aedes species. The B.t. israelensis toxins are most effective against Aedes but are also effective against Anopheles and Simulium. Between them these species are the vectors of Lymphatic Filarial Disease, Malaria, Dengue fever, Onchocerciasis and some forms of Encephalitis. Other insect species, animals, fish and plants are unaffected by the toxins. This is in marked contrast to the commonly used chemical insecticides which are non-specific and can enter the food chain and are becoming increasingly environmentally unacceptable.

The following Example illustrates the invention. In the accompanying drawings:

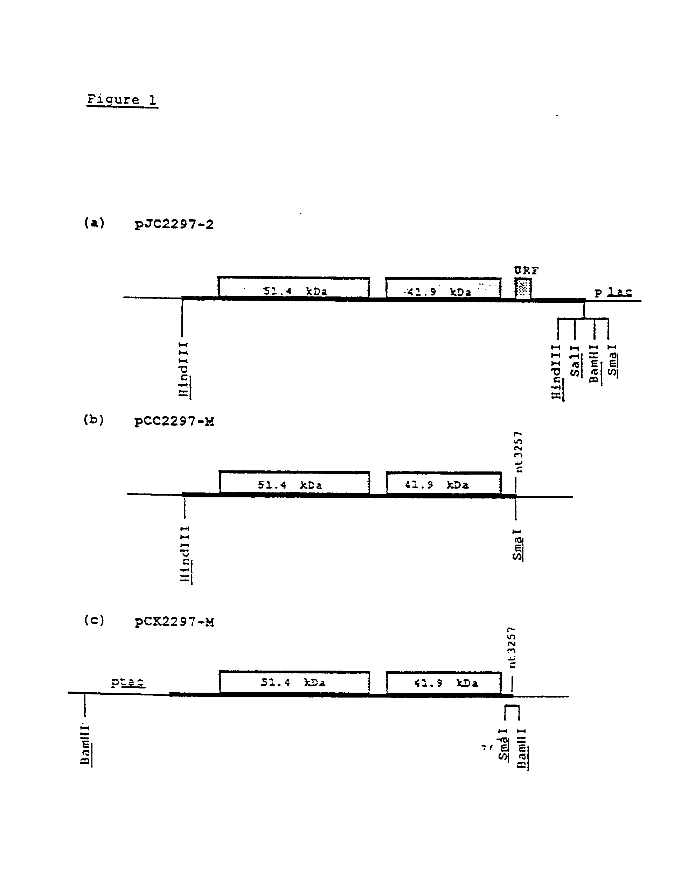

Figure 1 shows maps of the B. sphaericus 2297 toxin gene clones used in this work. (a) 4.2kb HindIII fragment in pUC12 (b) 3.25kb "M" fragment in pUC12 (c) 3.25kb "M" fragment in pKK223-3. Nucleotide number refer to the B. sphaericus 2297 sequence in SEQ ID NO: 1. Boxed in regions show the coding sequences.

: B. sphaericus derived sequence.           : Plasmid vector sequence.

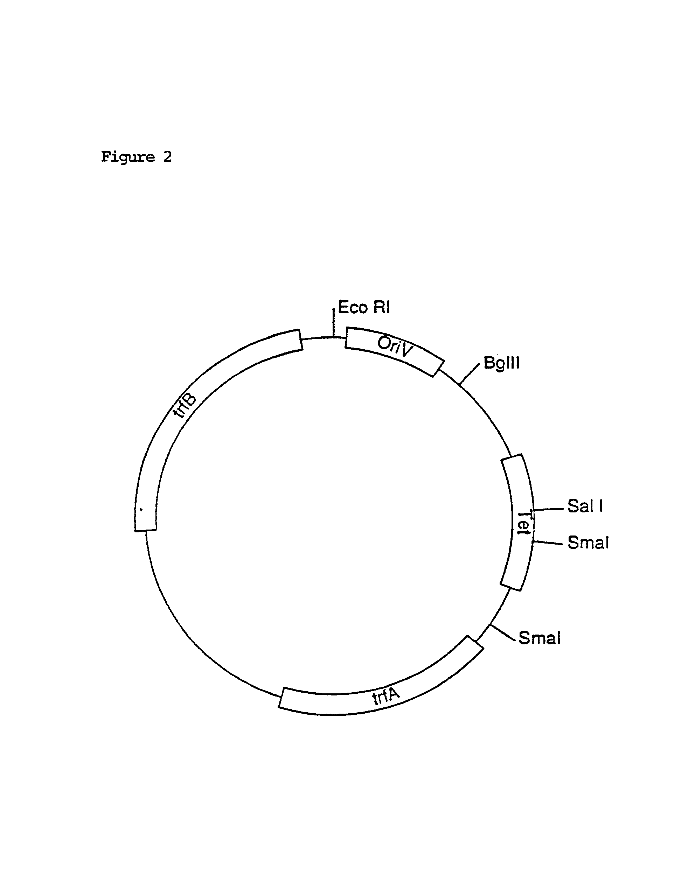

Figure 2 provides a physical and genetic map of pRK248 (Thomas et al, J. Bact. 141, 1, 213-222, 1980). OriV represents the origin of replication and trfA and B code for trans-acting replication functions. Tet codes for tetracycline resistance. All the recombinants described in the text were made by insertions into the EcoRI site.

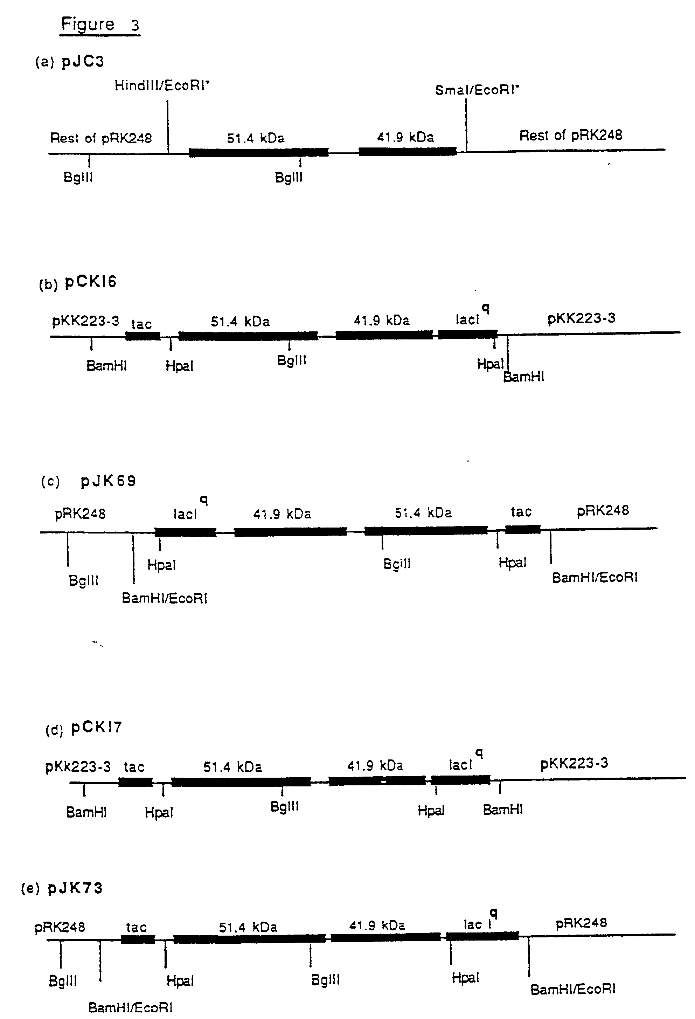

Figures 3 to 6 provide diagrams showing the structures of the different recombinant plasmids used in Exam-

ples 1 to 4 respectively.

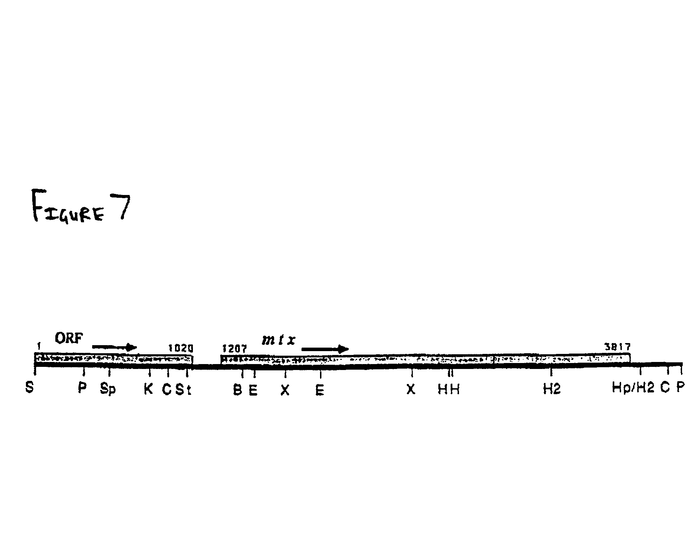

Figure 7 is a restriction map of the nucleotide sequence of the Reference Example. Shaded boxes represent the mtx (mosquitocidal toxin) gene and upstream open reading frame (ORF). Arrows show gene orientation. Nucleotide numbers correspond to those shown in SEQ ID NO: 5, beginning at the first nucleotide of the Sau3AI site. Restriction enzymes: S, Sau3AI (not unique); P, PstI; Sp, SphI; K, KpnI; C, ClaI; St, StuI; B, BamHI; E, EcoRI; X, XmnI; H, HindIII; H2, HindII; and Hp, HpaI.

## EXAMPLE 1

### Cloning B. sphaericus toxin genes into pUC12

The plasmid pJC2297-2 was obtained following ligation of an approximately 4.2kb HindIII fragment from B. sphaericus 2297 DNA into the E. coli vector pUC12. The cloned DNA included the genes encoding both the 51.4 and 41.9kDa toxin proteins together with a downstream unidentified reading frame (URF) and other flanking sequences [Fig. 1(a); Hindley and Berry, Nucl. Acids Res. 16, 4168, 1988; SEQ ID NOS: 1 to 4, SEQ ID NOS: 2 to 4 being the deduced amino acid sequences of the 51.4kDa protein, the 41.9kDa protein and the protein encoded by the URF respectively.

Experimental evidence implies that the two proteins, of 448 and 370 amino acids respectively, are translated from a single polycistronic mRNA which terminates approximately 130nt downstream of the stop codon for the latter protein (Hindley and Berry, Molec. Microbiol. 1, 187, 1987). The two coding sequences are separated by an untranslated region of 173nt. There is a further untranslated region which extends more than 400nt upstream of the initiator ATG for the 51.4 kDa protein. The probable ribosome binding sites are found 11 and 10 nt respectively upstream of the initiator ATG codons. Most of the DNA sequence data and discussion of its features have been presented elsewhere (Hindley and Berry, 1987; Baumann et al., J. Bacteriol. 170, 2045, 1988). The 4.2kb sequence (SEQ ID NO: 1) contains all the obvious Bacillus ancillary elements required for transcription and translation of the operon.

Cloning of the HindIII fragment into the multiple cloning site of pUC12 followed by transformation into E. coli JM105, yielded recombinants in which the toxin genes were expressed (unpublished data). One such recombinant pJC2297-2, in which the 4.2 kb HindIII fragment was cloned into the corresponding site in pUC12, is shown diagrammatically in Fig. 1(a). Other recombinants, in which the HindIII fragment was inserted in the opposite orientation, also expressed the toxin genes with similar efficiency. It can be concluded that the B. sphaericus promoter, or some related element, is recognised by the E. coli RNA polymerase. Baumann and Baumann (1988) have suggested that the region downstream of nt 400 may contain the B. sphaericus promoter. It is also apparent that the ribosome binding sites of the Bacillus mRNA are functional in E. coli. Where the orientation of the HindIII fragment is the opposite to that shown in Fig. 1(a), it is possible that transcription is also occurring from a lac promoter.

The transcript for the toxin appears to terminate at about nt 3260 (Hindley and Berry, 1987), i.e. approximately 130 nt downstream of the sequence coding for the 41.9kDa protein. Deletion of sequences downstream of this site should not affect the integrity of the transcriptional unit. This was confirmed by deleting approximately 1000 nt, up to nt 3257 (SEQ ID NO: 1) from the 3'-end of the HindIII fragment in pJC2297-2. For this, the plasmid pJC2297-2 was first digested with BamHI and the site protected with alpha-phosphorothioate deoxynucleotides using Klenow polymerase. This was followed by a second digestion with SalI and sequential treatment with exonucleaseIII, S1 nuclease and T4 DNA ligase, essentially as described by Henikoff (Gene 28, 351-359, 1984).

This produced a series of deletions, the extent of which were determined by sequencing. One such derivative, pCC2297-M contained the genes for both the 51.4 and 41.9kDa proteins and the transcriptional termination signal but had lost further downstream sequences [Fig. 1(b)]. When pCC2297-M was transformed into E. coli JM105, the larvicidal toxin was expressed, as demonstrated by in vivo toxicity to Culex larvae. It can be concluded that expression of the small URF and other sequences downstream of nt 3257 are not necessary for toxicity. E. coli JM105 harbouring pCC2297-M were deposited at the National Collection of Industrial and Marine Bacteria, Aberdeen, GB on 9 April 1990 under accession number NCIMB 40276.

### Recloning into the expression vector pKK223.3

A 3.25kb fragment was excised from pCC2297-M using SmaI and HindIII. It was blunt ended by filling in using Klenow polymerase and ligated into SmaI cut, phosphatase treated pKK223-3 (Brosius and Holy, Proc. Natl. Acad. Sci. USA 81, 6929, 1984; available from Pharmacia Ltd, GB) generating pCK2297-M Fig. 1(c). In this recombinant, the inserted coding sequence is located downstream of the strong tac promoter and the lac uv5 ribosome binding site. DNA sequencing across the fusion was used to check the polarity of the insertion and confirm its 5'-sequence. Thus pCK2297-M contained the insert from pCC2297-M recloned into the Smal site of pKK223-3.

Toxin gene coding fragments used for subsequent cloning into broad host range plasmids were the 3.25kb HindIII/Smal fragment from pCC2297-M described above, and an approximately 3.5kb BamHI fragment from pCK2297-M containing both toxin genes and the tac Promoter.

Cloning insecticidal toxin genes into pRK248

pRK248 is a TcR derivative of the broad host range IncPα plasmid, RK2 (Meyer et al. Mol. Gen. Genet. 152, 129-135, 1977) in which the regions encoding the conjugal transfer function have been deleted together with the ApR and KmR genes and other non-essential regions (Thomas et al J. Bact. 141, 1, 213-222, 1980).

All the cloning in pRK248 was carried out at the EcoRI site (Fig. 2) after blunt ending the EcoRI site with Klenow fragment. pCC2297-M (containing the B. sphaericus promoter) was digested with HindIII and Smal. The 3.25kb fragment was isolated from LMP agarose gel by phenol extraction. The cohesive ends produced by HindIII were blunted with Klenow fragment and the insert ligated into pRK248. After restriction analysis one clone pJC3 (Fig. 3a) was chosen for transformation into Caulobacter crescentus CB15 (Poindexter, Bacteriol. Revs. 28, 231-295, 1964; available from ATCC, US).

Caulobacter cells were prepared for electroporation by the method described by Dower et al (Nucl. Acids. Res. 16, 13, 5127, 1988) for E. coli. CB15 cells were grown in 1 litre of PYE medium (0.2% peptone, 0.1% yeast extract) supplemented with 0.02% MgSO₄7H₂O at 30°C until ABS₆₀₀0.7. The cells were collected after chilling for 15 min on ice, and centrifuging in a cold rotor at 6000 rpm for 15 min. The cells were resuspended in 1 litre of cold water and pelleted as before. The cells were resuspended in 20 ml 10% glycerol and the cells pelleted as above. Finally the cells were resuspended in 20 ml of 10% glycerol. The cells were stored at -70°C.

Electroporation (electrotransformation) was carried out using the BIO-RAD Gene Pulser with the pulse controller using the following settings: Conditions for electroporation:

Medium 10% glycerol
Sample volume 40μl
Temperature 2-4°C
Electrode gap 0.2 cm
Gene pulser settings
Voltage 2.5 kv
Capacitor 25 μF
Pulse controller settings 200 ohms

Cells were thawed and 1-2 μl DNA was added to 40 μl cells, mixed and left on ice for 0.5-1 min. The cells and DNA mixture was transferred to a cold 0.2 cm electroporation cuvette. After a single pulse at the above settings 1 ml of PYE medium was added and the cells allowed to regenerate at 30°C for 1 hr. The cells were plated onto PYE-agar containing tetracycline (2 μg/ml) and Nalidixic acid (10 μg/ml). Transformants were obtained.

The 3.5 kb BamHI fragment from pCK2297-M [Fig. 1(c)] was isolated and ligated into pRK248 after the cohesive BamHI ends were blunted by filling with Klenow fragment. Transformation into E. coli JM109 followed by selection on tetracycline plates gave recombinants which were toxic to Culex larvae (data not shown). However electrotransformation into C. crescentus failed to yield any transformants. A probable reason for this was that high level non-regulated expression of the toxin genes from the tac promoter was lethal to the Caulobacter.

In order to regulate transcription from the tac promoter, the lacIQ repressor gene was inserted distal to the toxin coding sequence. To do this, the lacIQ gene was isolated from pMJR1560 (Stark, Gene 51, 255-267, 1987) following digestion with KpnI and PstI and the 1.1 kb fragment purified following electrophoresis in LMP-agarose. After blunt-ending the fragment was cloned into the Smal site of pCK2297-M [Fig. 1(c)] yielding recombinants containing the lacIQ gene in both possible orientations (pCK16 and pCK17, Figure 3(b and d)). Both plasmids were digested with BamHI, the 4.6kb fragments isolated and, after blunt-ending, ligated into blunt-ended EcoRI linearized pRK248. Recombinant plasmids were analysed by restriction mapping. Two different plasmids were obtained viz, pJK69 and pJK73 (derived from pCK16 and pCK17 respectively) Fig. 3(c and e). These were transformed in C. crescentus CB15 as described above.

Toxicity Assays

The larvicidal activities of Caulobacter CB15 containing recombinant plasmids were assayed on second or third instar larvae of laboratory reared Culex quinquefasciatus. Caulobacter harbouring pRK248 recombinant and specifies resistance to streptomycin and sulphonamide (Scholz, P. et al (1989) GENE, 75:271-288). It can be mobilised across a wide variety of gram-negative bacterium in the presence of conjugative plasmids. In the current experiments RSF1010D1 rather than RSF1010 was used. RSF1010D1 is a spontaneous high copy number and mobilisation minus mutant of RSF1010 and it lacks the sequence 3098 to 3220 of RSF1010.

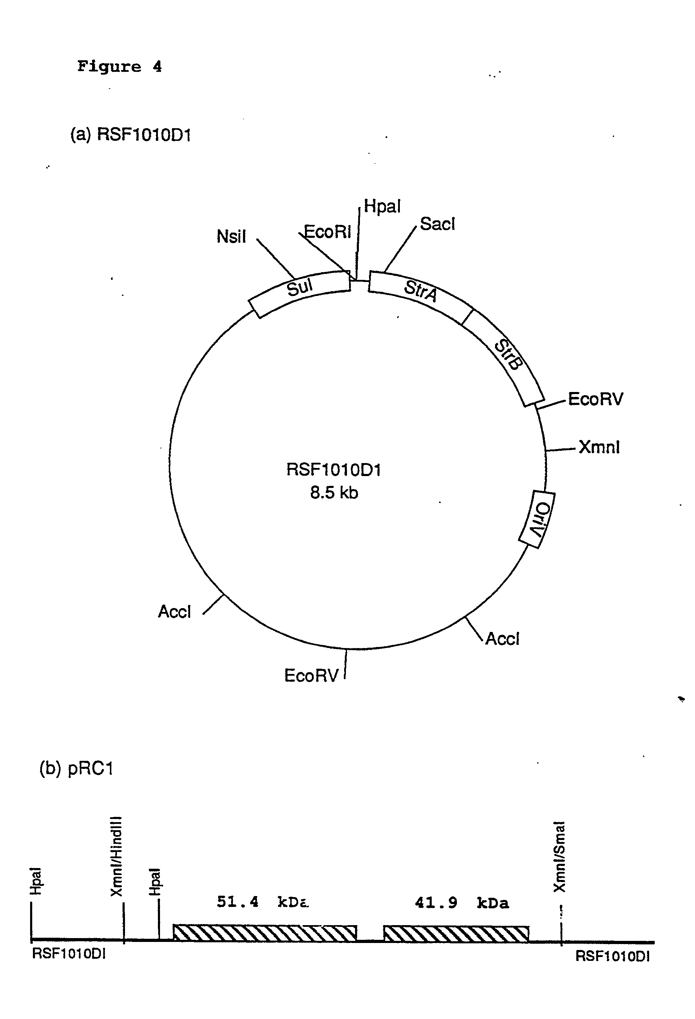

The toxin genes were inserted at the XmnI site (Fig. 4(a)) of RSF1010D1. The plasmid was first restricted with XmnI and phosphatase treated to prevent self ligation. pCC2297-M (Fig. 1(b)) (containing the natural B.sphaericus promoter and the genes encoding 51.4 and 41.9 kDa toxins) was restricted with HindIII and SmaI. The 3.25kb fragment containing the toxin genes was isolated following electrophoresis from LMP agarose gel by phenol extraction. The cohesive ends produced by HindIII were blunted with DNA polymerase 1 (Klenow fragment) and the insert ligated into the XmnI site of RSF1010D1. After restriction analysis one clone pRC1 (Fig. 4(b)) was chosen for electroporation into Caulobacter cresentus CB15. Electroporation was carried out as described previously and plated on PYE agar containing Streptomycin (25 μg/ml) and Nalidixic acid (10 μg/ml).

## Stability of pJC3 vs pRC1

Caulobacter cresentus CB15 carrying pJC3 (pRK248 recombinant with the HindIII - SmaI fragment of pCC2297-M, Fig. 3(a)) was grown in 25ml PYE medium with tetracycline (2μg/ml) while Caulobacter cresentus CB15 carrying pRC1 was grown in 25 ml PYE medium with Streptomycin (25 μg/ml) to produce a stationary culture (48 hrs). One ml of these cultures were transferred to 25 ml of PYE medium without any antibiotics and grown for 24hrs at 30°C. After 24 hrs of growth 1 ml was transferred to 25 ml of fresh PYE medium without any antibiotics. Subsequently every day 1ml of the overnight cultures were transferred to 25 ml fresh PYE medium without any antibiotics. 5 ml of the overnight cultures were harvested and washed with 0.85% saline and resuspended in 1ml of 0.85% saline. 0.5ml of this cell suspension was added to a cup containing 10 ml of water and 10 2nd instar Culex larvae. Mortality was recorded at 24 hrs. 100% mortality was observed for the pJC3 for 3 days without antibiotics while pRC1 showed no toxicity after 1 day without antibiotic selection to maintain the plasmid.

## EXAMPLE 3

### Expression in Caulobacter cresentus CB15 of a gene encoding a 130 kDa toxin from Bacillus thuringiensis var. israelensis

Bacillus thuringiensis var. israelensis (B.t.i) is a gram positive spore forming bacterium. It produces several δ-endotoxins which are highly toxic to mosquito and black fly larvae (Goldberg and Margalit 1977 Mosquito News 37:353-358). It has been shown that a 130 kDa protein alone is sufficient for toxicity to Aedes aegypti (Chanan Angsuthanasombat et al 1987 Mol. Gen. Genet. 208:384-389).

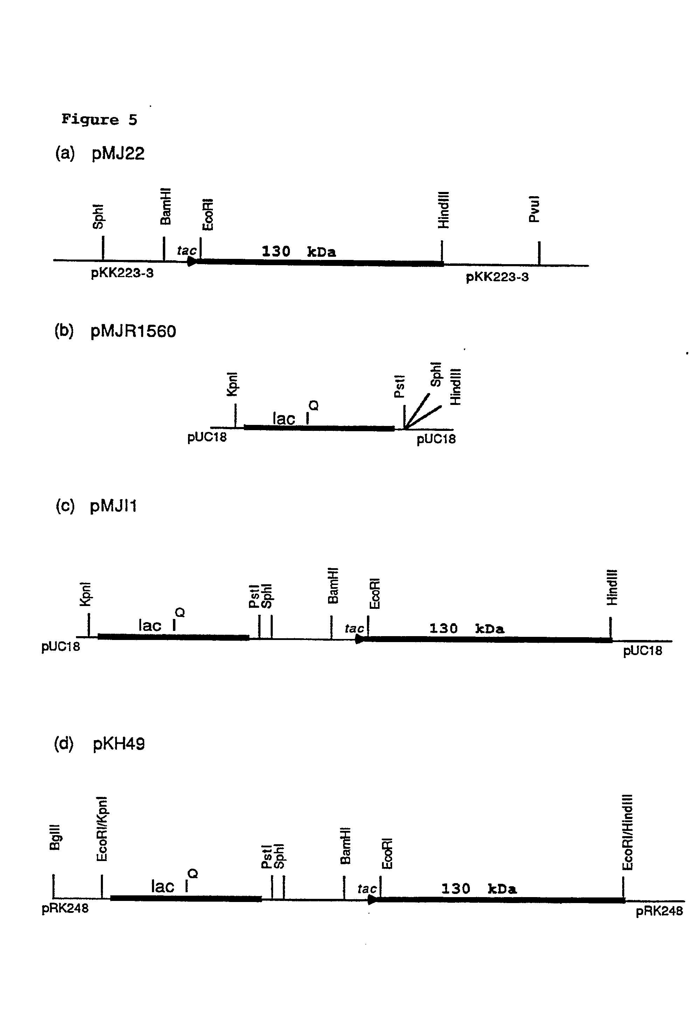

Two primers were designed to amplify the desired DNA fragment by Polymerase Chain Reaction (PCR) from total genomic DNA isolated from B.t.i. The sequence of the 5'-primer is shown in SEQ ID NO:8. That for the 3'-primer is shown in SEQ ID N0:9. The primers were based on the sequence of Sumalee Tungpradubkul et al. (1988) (Nucleic Acids Res 16(4): 1637-1638) and the 5' end primer was designed such that the EcoRI site internal to the structural gene for the 130 kDa protein was eliminated by a silent mutation. The PCR product was restricted with EcoRI and HindIII and the fragment containing the toxin gene was cloned into pKK223-3 (E. coli expression vector). Among the recombinants a clone designated pMJ22 (Fig. 5(a)) was obtained in which the gene encoding the B.t.i. 130 kDa toxin is under the control of the inducible tac promoter. This clone was found to be toxic to Aedes aegypti in E. coli when induced with IPTG (2mM).

When cloning the genes encoding the B. sphaericus 51.4 and 41.9 kDa toxins under the control of the tac promoter in Caulobacter the presence of the lac IQ repressor was found to be essential. pMJ22 was restricted with SphI and HindIII and the fragment containing the toxin gene and the tac promoter was isolated and was cloned into pMJR1560 (Stark, 1987; Fig. 5(b)) giving rise to pMJI1 (Fig. 5(c)). This plasmid in E. coli was also found to be toxic to Aedes aegypti when induced with IPTG (2mM).

Recombinant plasmid pMJI1 was restricted with KpnI and HindIII and the fragment containing lacIQ and the gene encoding the B.t.i. 130 kDa toxin gene was isolated and purified from LMP agarose. The cohesive end of the KpnI site was blunt ended with T4 DNA polymerase and the cohesive end of the HindIII site was blunt ended with Klenow DNA polymerase. This blunt ended DNA fragment was cloned into pRK248 EcoRI blunt ended site. pRK248 is a TcR derivative of the broad host range IncP plasmid, RK2 (Meyer et al. Mol. Gen. Genet. 152, 129-135, 1977). The resulting recombinant clone pKH49 (Fig. 5(d)) was found to be toxic E. coli (100% at 48 hrs) to Aedes aegypti larvae. This plasmid clone was electoporated into Caulobacter cresentus plasmids were grown in PYE medium (0.2% peptone, 0.1% yeast extract) for 48 hrs at 30°C in the presence of Tetracycline (2 μg/ml) and Nalidixic Acid (10 μg/ml). Cells from 5 ml of the culture were harvested by centrifugation, washed in 0.85% saline and resuspended in 1 ml 0.85% saline. Aliquots were added to 10 larvae in a plastic cup containing 10 ml water. Mortality was recorded after 24 and 48 hrs. Controls were fed with 0.5 ml suspension of Caulobacter containing the non-recombinant pRK248. In order to prevent starvation, the volume of the aliquots of the toxin-expressing Caulobacter cells was always adjusted to 0.5 ml with the control Caulobacter suspension. For induction of toxin gene expression, cells harbouring pJK69 and pJK73 were grown in 5 ml PYE for 48 hrs and harvested as above and resuspended in 5 ml PYE containing 5mM IPTG followed by incubation for 1 hr at 30°C. The cells were spun down, washed and resuspended in 1 ml 0.85% saline. The results are shown in Table 1.

## Table 1: Toxicity Assays

| Volume of Caulobacter suspension added to 10ml water | No. of survivors after 24 hrs | | No. of survivors after 48 hrs | |
|---|---|---|---|---|
| **Caulobacter CB15, carrying pJC3** | | | | |
| 500μl | 0 | 0 | 0 | 0 |
| 250μl | 0 | 0 | 0 | 0 |
| 100μl | 0 | 0 | 0 | 0 |
| 50μl | 5 | 5 | 5 | 5 |
| 10μl | 9 | 10 | 9 | 10 |
| 0μl | 10 | 10 | 10 | 10 |
| **CB15 with pJK69 (uninduced)** | | | | |
| 50μl | 0 | 0 | 0 | 0 |
| 10μl | 0 | 0 | 0 | 0 |
| 1μl | 6 | 6 | 6 | 6 |
| **CB15 with pJK69 (induced)** | | | | |
| 50μl | 0 | 0 | 0 | 0 |
| 10μl | 0 | 0 | 0 | 0 |
| 1μl | 3 | 2 | 3 | 2 |
| **CB15 with pJK73 (uninduced)** | | | | |
| 50μl | 0 | 0 | 0 | 0 |
| 10μl | 0 | 0 | 0 | 0 |
| 1μl | 10 | 10 | 9 | 10 |
| **CB15 with pJK73 (induced)** | | | | |
| 50μl | 0 | 0 | 0 | 0 |
| 10μl | 0 | 0 | 0 | 0 |
| 1μl | 7 | 7 | 6 | 5 |

EXAMPLE 2

Expression of Bacillus sphaericus 2297 toxin genes in Caulobacter cresentus CB15 using broad host range vector RSF1010D1

RSF1010 is a small non-conjugative broad host range plasmid. It belongs to the Inc Q group of plasmids CB15 and plated on PYE plates containing 2μg/ml tetracycline and 10ug/ml Nalidixic acid.

Individual Caulobacter transformants were grown in 5ml PYE medium containing the antibiotics Tetracycline (2μg/ml) and Nalidixic acid (10μg/ml) for 48 hrs. The cells were harvested by centrifugation and resuspended in 5ml of the same medium containing 5mM IPTG to induce expression of the 130 kDa toxin and incubated at 30°C with shaking for 5hr. The cells were harvested as above and washed once with 0.85% saline and resuspended in 1ml of 0.85% saline. 1ml of this suspension was added to 10ml water containing 10 2nd instar Aedes aegypti larvae. Toxicity was recorded at 24hr and 48hr. At 24hr there was no mortality but at 48hr there was 30% mortality compared to controls.

## EXAMPLE 4

### Expression of B.sphaericus SSII-1 100 kDa toxin in Caulobacter cresentus CB15 using broad host range vector pRK248

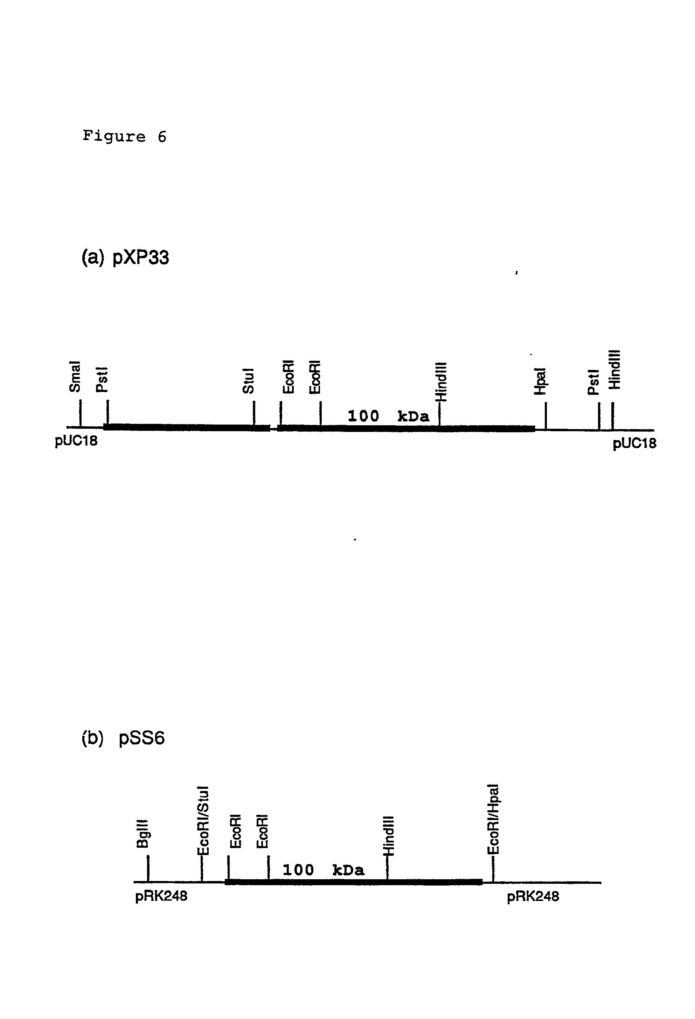

The gene encoding the 100 kDa toxin from B.sphaericus SSII-1 was identified from a cosmid library of 250 clones (Reference Example). The plasmid pXP33 (Fig 6(a)) was obtained following ligation into pUC18 of a 3.8kb PstI fragment derived from the cosmid clone designated pKF2. This DNA fragment encodes the 100kDa toxin.

The plasmid pXP33 was restricted with StuI and HpaI and after electrophoresis the 2.9kb fragment containing the toxin gene was isolated from LMP agarose gel by phenol extraction. This fragment was ligated into the blunt ended EcoRI site of pRK248. After restriction analysis one clone designated pSS6 (Fig. 6(b)) was selected and electroporated into Caulobacter cresentus CB15 cells as described previously. The Caulobacter cells harbouring PSS6 were assayed for toxicity towards both Culex and Aedes larvae. The Caulobacter transformants showed 30% toxicity towards Culex larvae after 48hrs while no toxicity was detected towards Aedes larvae.

### REFERENCE EXAMPLE

### Cloning and sequencing of the gene encoding a 100kDa mosquitocidal toxin from Bacillus sphaericus SSII-1

#### 1. Materials and Methods

Construction of the cosmid library. To prepare total genomic DNA from B.sphaericus SSII-1 (Singer, 1973, Nature, 244, 110-111), we lysed cells in the presence of 1% sodium dodecyl sulfate and 0.1 mg of proteinase K per ml. DNA was purified from the lysate by the method of Ausubel et al. (Current protocols in molecular biology, vol. I, 1987, Wiley Interscience, New York, US). In brief, hexadecyltrimethyl ammonium bromide was added and the mixture was incubated at 65°C for 20 min prior to extraction with chloroform isoamyl alcohol. DNA was precipitated with ethanol from the aqueous phase and purified by banding on a cesium chloride gradient containing ethium bromide. DNA was partially digested with BamHI under conditions found to yield a predominance of fragments in the range of 20 to 40 kb. Typically, 0.12 U of enzyme was used for each microgram of DNA in a 14 min incubation at 37°C. Fragments from the partial digest were ligated into Bam HI-cut phophatase-treated cosmid pHC79 (Hohn and Collins, 1980, Gene 11, 291-298). These cosmids were packaged with the Packagene in vitro packaging system (Promega) in accordance with the manufacturer's instructions, transfected into E. coli HB101, and plated onto LB agar containing 60μg of ampicillin per ml.

Isolation of toxic clones. Two hundred fifty colonies were individually picked and stored. Pooled groups of 10 colonies each were grown overnight and assayed for toxicity. Ten second-instar C. quinquefasciatus larvae were placed in 5ml of water, to which the cells for assay were added. Mortality was assessed after 24 h of incubation at 30°C. One pool exhibited toxicity. The individual clones in this pool were separately assayed against C. quinquefasciatus larvae. A single, toxin-producing clone, designated pKF2, was identified and used as the parent clone for the experiments.

Subcloning of the toxin-encoding fragment. Subclones were produced from cosmid clone pKF2 to focus on the region of the inserted DNA encoding toxicity. At each step, subclones were assayed for larvicidal activity, and those exhibiting toxicity were chosen for further manipulation.

Cosmid clone pKF2 was subjected to partial digestion with Sau3AI (0.2μg of cosmid DNA, 0.1 U of Sau3AI, 15 min, 37°C), and the resulting fragments were ligated into BamHI-cut, phosphatase-treated pUC18. Four toxic clones were produced, and restriction mapping revealed that they contained overlapping inserts. The plasmid designated pKG19 contained the shortest inserted sequence (approximately 9 kb) and was chosen for further subcloning. This plasmid was digested with XbaI to remove an approximately 4.5 kb fragment. Religation of the remaining insert yielded clone pKG19X, which retained full toxicity. A Pstl fragment of 3.8 kb derived from pKG19X was recloned into Pstl-cut, phosphatase-treated pUC18. Two toxic clones, pXP33 and pXP34, which contained the same Pstl fragment in opposite orientations relative to the pUC18 vector were produced.

DNA sequencing. Double-stranded DNA sequencing was carried out by the method of Heinrich (Guidelines for quick and simple plasmid sequencing, 1986, Boehringer GmbH, Mannheim, DE) with plasmid DNA as the template. Subclones from plasmids pXP33 and pXP34 were made in pUC18 with the restriction sites shown in Fig. 7. A sequence further upstream was obtained from clone pKG19 (nucleotides (nt) 1 to 312). DNA was sequenced with the pUC-M13 forward and reverse primers. In addition, further oligonucleotide primers were synthesized to ensure complete sequencing of both strands.

## 2. Results

Features-of the sequence. SEQ ID NO. 5 shows a sequence of 4133 nt containing two open reading frames along with the deduced amino acid sequence for these open reading frames. The first open reading frome is the 3' end of a longer open reading frame. The second encodes the 100kDa toxin of B. sphaericas SSII-1. SEQ ID NO. 6 presents the amino acid sequence of the first open reading frame. SEQ ID NO:7 presents the amino acid sequence of the 100kDa toxin. A restriction map of the nucleotide sequence is shown in Figure 7. The nucleotide sequence has been submitted to the GenBank database (nucleotide sequence accession number M60446).

Sequence of the toxin. The toxicity of recombinants containing sequences corresponding to nt 960 to 4133 (Stul-Pstl) and nt 307 to 3864 (Pstl-Hpal) indicates that the open reading frame commencing at nt 1207 and terminating at nt 3817 represents the toxin gene. We have designated this gene the mtx (mosquitocidal toxin) gene. This gene encodes a protein of 100.557 kDa and 870 amino acids in length.

The deduced protein has features characteristic of signal peptides of gram-positive bacteria, namely, (i) positively charged residues in the N terminus followed by (ii) a stretch of residues with a neutral or hydrophobic character and (iii) a cleavage site (often after an alanine residue) at the C terminus of the signal sequence. The N terminus of the deduced protein contains three lysine residues, an isoleucine-rich stretch of neutral and hydrophobic residues, and a potential cleavage site following the alanine residue at position 30, forming a potential signal sequence. However, the toxic activity of our SSII-1 cultures was found to be associated with the cell pellet and not with the medium.

Toxicity of the E. coli clones. Serial 10-fold dilutions of E. coli containing clones pXP33 and pXP34 were assayed for toxicity for second-instar larvae of the mosquitoes C. quinquefasciatus and Aedes aeqypti. Both clones were toxic for both species of mosquitoes, causing 100% mortality in C. quinquefasciatus at minimum cell concentrations of 2 x 10⁶ cells per ml at 24 h and 2 x 10⁵ cells per ml at 48 h and in A. aeqypti at minimum cell concentrations of 2 x 10⁷ cells per ml at 24 h and 2 x 10⁶ cells per ml at 48 h. The minimum concentrations of B. sphaericus SSII-1 cells necessary to produce 100% mortality were found to be 1/10 the concentrations of the recombinants in every case, possibly because of lower expression in the recombinants or the presence of other, unidentified toxins in strain SSII-1.

The toxicity of B. sphaericus SSII-1 declined rapidly upon storage at 4°C. The same loss of toxicity was observed with E. coli transformed with our initial cosmid clone, pKF2. However, greater stability was observed in toxins produced by later subclones derived from pKF2. The toxicity of these subclones was maintained for at least 3 weeks at 4°C.

## Claims

1. Caulobacter which express an insecticidal protein.

2. Caulobacter according to claim 1, in which the protein is an insecticidal toxin of a strain of Bacillus or a modified insecticidally-active form of the toxin.

3. Caulobacter according to claim 2, in which the insecticidal toxin is a Bacillus thuringiensis toxin or Bacillus sphaericus toxin.

4. Caulobacter according to any one of the preceding claims, in which a gene encoding the protein is provided under the control of a heterologous promoter.

5. Caulobacter according to any one of the preceding claims, in which a repressor gene is provided to regulate the level of expression of the protein.

6. A process for the preparation of Caulobacter according to claim 1, which process comprises transforming a strain of Caulobacter with a gene encoding the insecticidal protein such that the protein is expressed by the transformed Caulobacter strain.

7. A process according to claim 6, in which a strain of Caulobacter is transformed by a plasmid vector which contains the insecticidal protein gene.

8. A process according to claim 6, in which a strain of Caulobacter is transformed by homologous recombination whereby a DNA fragment encoding the insecticidal toxin gene(s) is integrated into the chromosomal DNA of the Caulobacter.

9. An insecticidal composition which comprises a carrier or diluent and, as active principle, Caulobacter as claimed in any one of claims 1 to 5 or which have been prepared by a process as claimed in any one of claims 6 to 8.

10. A method of controlling insects which have an aquatic phase in their life cycle, which method comprises applying to the surface of water at a selected locus Caulobacter as claimed in any one of claims 1 to 5 or which have been prepared by a process as claimed in any one of claims 6 to 8.

11. A method according to claim 10, wherein the locus is a larval feeding zone.

## Figure 1

(a)    pJC2297-2

(b)    pCC2297-M

(c)    pCK2297-M

Figure 2

## Figure  3

(a) pJC3

(b) pCKI6

(c)  pJK69

(d) pCKI7

(e) pJK73

**Figure 4**

(a) RSF1010D1

(b) pRC1

Figure 5

(a) pMJ22

(b) pMJR1560

(c) pMJI1

(d) pKH49

Figure 6

(a) pXP33

(b) pSS6

FIGURE 7

(1) INFORMATION FOR SEQ ID NO:1:

    (i) SEQUENCE CHARACTERISTICS:
      (A) LENGTH: 3458 base pairs
      (B) TYPE: nucleic acid
      (C) STRANDEDNESS: double
      (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:
      (A) ORGANISM: Bacillus sphaericus
      (B) STRAIN: 2297

    (ix) FEATURE:
      (A) NAME/KEY: CDS
      (B) LOCATION: 499..1842

    (ix) FEATURE:
      (A) NAME/KEY: CDS
      (B) LOCATION: 2019..3128

    (ix) FEATURE:
      (A) NAME/KEY: CDS
      (B) LOCATION: 3357..3398

    (ix) FEATURE:
      (A) NAME/KEY: RBS
      (B) LOCATION: 484..488

    (ix) FEATURE:
      (A) NAME/KEY: RBS
      (B) LOCATION: 2001..2009

    (xi) SEQUENCE DESCRIPTION: SEQ ID NO:1:

```
AAGCTTGTCA ACATGTGAAG ATTAAAGGTA ACTTTCAGTT TCTTTCTGTG TAACAATACA    60

CGAAGTAATA TATGTATTTC TATAGAAATT AATTAAAAAA AGACCTAGTG AACTGCACCC   120

TGTCAAGTAG ATAGTGGAAA TAATAAAAAT GTATTAAGCG GCTTGTGCTC TTAATTCTAA   180

GAAGGTGATG AAAAACTTGC ACTCTAGGGC GTACCAAGCA ATCAAAGGAG AAACGTTGTA   240

TCTCCGGATT AATTCGAACT ACAACGTCTA TTGTAGATTA AACCGTAAGA GATTATATAA   300
```

11

```
TTATTATAAC AATACTTTAA TTAATTCAAT TGAAATTAAA CAAACAACTT TTTCGGTATA        360

TACTATCTAA CATATCGGTT TCAGTCCCAT CGTTCTAAAG GTACCTTCTT TTTGGTTAAC        420

GTTATTTAAT GAACTTTTTA GGTTTTAAAT AATATAATGA GAAGTATTTT TTATCAATGA        480

TAAGGAGATG AAGAAAGC ATG TGC GAT TCA AAA GAC AAT TCT GGC GTT TCA         531
                     Met Cys Asp Ser Lys Asp Asn Ser Gly Val Ser
                      1               5                    10

GAA AAA TGC GGA AAG AAA TTT ACT AAT TAC CCG CTA AAT ACT ACT CCT         579
Glu Lys Cys Gly Lys Lys Phe Thr Asn Tyr Pro Leu Asn Thr Thr Pro
             15                  20                  25

ACA AGC CTA AAT TAT AAC CTT CCA GAA ATA TCA AAA AAA TTT TAT AAC         627
Thr Ser Leu Asn Tyr Asn Leu Pro Glu Ile Ser Lys Lys Phe Tyr Asn
         30                  35                  40

CTT AAG AAT AAA TAT TCA CGG AAT GGT TAT GGT TTA TCA AAA ACC GAA         675
Leu Lys Asn Lys Tyr Ser Arg Asn Gly Tyr Gly Leu Ser Lys Thr Glu
         45                  50                  55

TTT CCT TCA AGT ATC GAA AAT TGC CCA TCT AAC GAA TAT TCA ATA ATG         723
Phe Pro Ser Ser Ile Glu Asn Cys Pro Ser Asn Glu Tyr Ser Ile Met
 60                  65                  70                  75

TAT GAT AAT AAA GAT CCT CGA TTC TTG ATT CGG TTT TTA TTA GAT GAT         771
Tyr Asp Asn Lys Asp Pro Arg Phe Leu Ile Arg Phe Leu Leu Asp Asp
                 80                  85                  90

GGT AGA TAT ATT ATT GCA GAT AGA GAC GAT GGA GAA GTT TTT GAT GAA         819
Gly Arg Tyr Ile Ile Ala Asp Arg Asp Asp Gly Glu Val Phe Asp Glu
             95                  100                 105

GCA CCT ACT TAT TTG GAT AAT AAC AAT CAC CCT ATC ATA AGT AGA CAT         867
Ala Pro Thr Tyr Leu Asp Asn Asn Asn His Pro Ile Ile Ser Arg His
         110                 115                 120

TAT ACC GGA GAA GAG AGA CAA AAG TTT GAG CAG GTA GGT AGT GGA GAT         915
Tyr Thr Gly Glu Glu Arg Gln Lys Phe Glu Gln Val Gly Ser Gly Asp
         125                 130                 135

TAT ATT ACG GGA GAG CAA TTT TTT CAA TTC TAT ACA CAA AAC AAA ACA         963
Tyr Ile Thr Gly Glu Gln Phe Phe Gln Phe Tyr Thr Gln Asn Lys Thr
140                 145                 150                 155

CGT GTA TTG TCA AAT TGT AGG GCG CTT GAC AGT AGG ACA ATA TTA CTA        1011
Arg Val Leu Ser Asn Cys Arg Ala Leu Asp Ser Arg Thr Ile Leu Leu
             160                 165                 170
```

```
TCT ACT GCA AAA ATC TTC CCA ATT TAC CCT CCA GCT TCT GAA ACT CAA        1059
Ser Thr Ala Lys Ile Phe Pro Ile Tyr Pro Pro Ala Ser Glu Thr Gln
            175             180             185

CTA ACA GCT TTC GTT AAT AGT TCA TTT TAT GCT GCG GCA ATT CCT CAA        1107
Leu Thr Ala Phe Val Asn Ser Ser Phe Tyr Ala Ala Ala Ile Pro Gln
            190             195             200

TTA CCC CAA ACA TCC TTA CTT GAG AAT ATT CCT GAG CCT ACT AGT CTC        1155
Leu Pro Gln Thr Ser Leu Leu Glu Asn Ile Pro Glu Pro Thr Ser Leu
            205             210             215

GAT GAT TCT GGA GTA TTA CCA AAA GAT GCA GTA AGA GCA GTT AAA GGA        1203
Asp Asp Ser Gly Val Leu Pro Lys Asp Ala Val Arg Ala Val Lys Gly
220             225             230             235

AGT GCG CTA TTA CCT TGT ATA ATA GTA CAT GAT CCT AAT TTA AAC AAT        1251
Ser Ala Leu Leu Pro Cys Ile Ile Val His Asp Pro Asn Leu Asn Asn
            240             245             250

TCC GAT AAA ATG AAA TTT AAT ACC TAC TAT CTT TTA GAA TAT AAA GAA        1299
Ser Asp Lys Met Lys Phe Asn Thr Tyr Tyr Leu Leu Glu Tyr Lys Glu
            255             260             265

TAC TGG CAT CAA TTA TGG TCA CAA ATT ATA CCT GCT CAT CAA ACT GTA        1347
Tyr Trp His Gln Leu Trp Ser Gln Ile Ile Pro Ala His Gln Thr Val
            270             275             280

AAA ATA CAG GAA CGA ACA GGA ATA TCT GAA GTT GTA CAA AAT AGC ATG        1395
Lys Ile Gln Glu Arg Thr Gly Ile Ser Glu Val Val Gln Asn Ser Met
            285             290             295

ATT GAA GAT TTA AAT ATG TAT ATT GGA GCA GAT TTT GGC ATG TAT TTT        1443
Ile Glu Asp Leu Asn Met Tyr Ile Gly Ala Asp Phe Gly Met Tyr Phe
300             305             310             315

TAT TTG AGA TCT AGC GGA TTT AAG GAA CAA ATA ACA AGG GGG CTA AAT        1491
Tyr Leu Arg Ser Ser Gly Phe Lys Glu Gln Ile Thr Arg Gly Leu Asn
            320             325             330

AGG CCT TTA TCC CAA ACG ACC ACT CAG TTA GGA GAA AGA GTA GAA GAA        1539
Arg Pro Leu Ser Gln Thr Thr Thr Gln Leu Gly Glu Arg Val Glu Glu
            335             340             345

ATG GAG TAT TAT AAT TCT AAT GAT TTG GAT GTT AGA TAT GTG AAA TAC        1587
Met Glu Tyr Tyr Asn Ser Asn Asp Leu Asp Val Arg Tyr Val Lys Tyr
            350             355             360

GCA TTG GCT AGA GAA TTC ACA CTA AAA CGC GTT AAT GGT GAA ATT GTA        1635
Ala Leu Ala Arg Glu Phe Thr Leu Lys Arg Val Asn Gly Glu Ile Val
            365             370             375
```

```
AAA AAT TGG GTT GCT GTA GAT TAT CGA ATG GCA GGT ATA CAA TCA TAT        1683
Lys Asn Trp Val Ala Val Asp Tyr Arg Met Ala Gly Ile Gln Ser Tyr
380             385             390             395

CCT AAT GCA CCT ATA ACT AAT CCA CTT ACG CTA ACA AAA CAT ACA ATT        1731
Pro Asn Ala Pro Ile Thr Asn Pro Leu Thr Leu Thr Lys His Thr Ile
            400             405             410

ATT CGA TGT GAA AAT AGT TAC GAT GGA CAC ATA TTT AAA ACA CCT TTA        1779
Ile Arg Cys Glu Asn Ser Tyr Asp Gly His Ile Phe Lys Thr Pro Leu
            415             420             425

ATC TTT AAA AAT GGT GAA GTT ATT GTA AAA ACG AAT GAA GAA TTA ATA        1827
Ile Phe Lys Asn Gly Glu Val Ile Val Lys Thr Asn Glu Glu Leu Ile
            430             435             440

CCT AAA ATT AAC CAG TGATACTCTT TAAATTCAAA TATTCATTAC CATGTTATTT        1882
Pro Lys Ile Asn Gln
            445

AAAATAGTAG ATAGATGAAA TAAATAGTAT AAATTAAGAC AACAACTTAA TTTTGACACA      1942

TAAGAATAAT TTTTAAATGT ATAAATAGTA TTTAGAGTGT TATTGCAATA TATTTTATGA      2002

AAGGGAGCTA AAAGAC ATG AGA AAT TTG GAT TTT ATT GAT TCT TTT ATA         2051
                  Met Arg Asn Leu Asp Phe Ile Asp Ser Phe Ile
                   1           5               10

CCC ACA GAA GGA AAG TAC ATT CGC GTT ATG GAT TTT TAT AAT AGC GAG        2099
Pro Thr Glu Gly Lys Tyr Ile Arg Val Met Asp Phe Tyr Asn Ser Glu
            15              20              25

TAT CCT TTC TGT ATA CAT GCA CCC TCA GCC CCT AAT GGG GAT ATT ATG        2147
Tyr Pro Phe Cys Ile His Ala Pro Ser Ala Pro Asn Gly Asp Ile Met
            30              35              40

ACA GAA ATC TGT AGC AGA GAA AAT AAC CAA TAT TTT ATT TTT TTT CCT        2195
Thr Glu Ile Cys Ser Arg Glu Asn Asn Gln Tyr Phe Ile Phe Phe Pro
            45              50              55

ACT GAT GAT GGT CGA GTA ATT ATT GCA AAT AGG CAT AAT GGG TCC GTT        2243
Thr Asp Asp Gly Arg Val Ile Ile Ala Asn Arg His Asn Gly Ser Val
            60              65              70              75

TTT ACC GGA GAA GCT ACA AGT GTA GTA TCA GAT ATC TAT ACT GGT AGC        2291
Phe Thr Gly Glu Ala Thr Ser Val Val Ser Asp Ile Tyr Thr Gly Ser
            80              85              90

CCA TTA CAG TTT TTT AGA GAG TTC AAA AGA ACT ATG TCA ACT TAT TAT        2339
Pro Leu Gln Phe Phe Arg Glu Phe Lys Arg Thr Met Ser Thr Tyr Tyr
            95              100             105
```

```
TTA GCG ATA CAA AAT CCT GAA TCC GCA ACA GAT GTG AGA GCT CTA GAA          2387
Leu Ala Ile Gln Asn Pro Glu Ser Ala Thr Asp Val Arg Ala Leu Glu
        110             115                 120

CCG AAT TCC CAT GAG CTG CCA TCT CGC CTC TAT TTC ACT AAC AAT ATT          2435
Pro Asn Ser His Glu Leu Pro Ser Arg Leu Tyr Phe Thr Asn Asn Ile
        125             130                 135

GAA AAT AAT AGC AAC ATA TTA ATT TCT AAT AAG GAA CAA ATA TAT TTA          2483
Glu Asn Asn Ser Asn Ile Leu Ile Ser Asn Lys Glu Gln Ile Tyr Leu
140             145                 150                 155

ACC TTG CCT TCT CTT CCA GAA AAC GAG CAA TAC CCT AAA ACT CCA GTA          2531
Thr Leu Pro Ser Leu Pro Glu Asn Glu Gln Tyr Pro Lys Thr Pro Val
                160             165                 170

TTA AGC GGT ATC GAT GAT ATA GGA CCT AAT CAA TCA GAG AAA TCA ATA          2579
Leu Ser Gly Ile Asp Asp Ile Gly Pro Asn Gln Ser Glu Lys Ser Ile
                175             180                 185

ATA GGA AGT ACT CTT ATC CCA TGT ATA ATG GTT TCG GAT TTT ATT AGT          2627
Ile Gly Ser Thr Leu Ile Pro Cys Ile Met Val Ser Asp Phe Ile Ser
                190             195                 200

TTG GGG GAG AGA ATG AAA ACG ACT CCA TAT TAT TAT GTA AAG CAC ACT          2675
Leu Gly Glu Arg Met Lys Thr Thr Pro Tyr Tyr Tyr Val Lys His Thr
        205             210                 215

CAA TAT TGG CAA AGC ATG TGG TCC GCG CTC TTT CCA CCC GGC TCT AAA          2723
Gln Tyr Trp Gln Ser Met Trp Ser Ala Leu Phe Pro Pro Gly Ser Lys
220             225                 230                 235

GAG ACA AAA ACT GAG AAA TCA GGT ATT ACT GAC ACT TCT CAA ATA AGT          2771
Glu Thr Lys Thr Glu Lys Ser Gly Ile Thr Asp Thr Ser Gln Ile Ser
                240             245                 250

ATG ACT GAC GGG ATT AAT GTT TCA ATC GGA GCA GAT TTC GGA TTA AAG          2819
Met Thr Asp Gly Ile Asn Val Ser Ile Gly Ala Asp Phe Gly Leu Lys
                255             260                 265

TTT GGA AAT AAA ACG TTT GGA ATT AAG GGG GGG TTC ACC TAT GAT ACA          2867
Phe Gly Asn Lys Thr Phe Gly Ile Lys Gly Gly Phe Thr Tyr Asp Thr
                270             275                 280

AAG ACT CAA ATA ACT AAT ACC TCC CAA TTG TTA ATA GAA ACA ACT TAT          2915
Lys Thr Gln Ile Thr Asn Thr Ser Gln Leu Leu Ile Glu Thr Thr Tyr
        285             290                 295

ACT AGA GAA TAC ACA AAT ACA GAA AAT TTT CCT GTT AGA TAT ACA GGC          2963
Thr Arg Glu Tyr Thr Asn Thr Glu Asn Phe Pro Val Arg Tyr Thr Gly
300             305                 310                 315
```

```
TAT GTT TTA GCG TCA GAA TTT ACT TTA CAT CGT AGT GAT GGA ACT CAG        3011
Tyr Val Leu Ala Ser Glu Phe Thr Leu His Arg Ser Asp Gly Thr Gln
            320             325             330

GTT AAT ACG ATC CCA TGG GTT GCT TTA AAC GAT AAC TAT ACA ACA ATA        3059
Val Asn Thr Ile Pro Trp Val Ala Leu Asn Asp Asn Tyr Thr Thr Ile
            335             340             345

GCA AGA TAT CCA CAT TTT GCA AGT GAA CCT TTA CTA GGA AAT ACA AAG        3107
Ala Arg Tyr Pro His Phe Ala Ser Glu Pro Leu Leu Gly Asn Thr Lys
            350             355             360

ATT ATT ACA GAT GAT CAA AAC TAAATTTAAA CAATATTCTT GAACTAATAG           3158
Ile Ile Thr Asp Asp Gln Asn
            365             370

ATGTTAAATA GAACAATTAA TAACAATTTA AGTACTTTTG GATTATAGTG AAGGGACCTA      3218

TAAGCATAGC TTTTAGGTCC CTTTTAAGTT GCTTTTTTTC GTTTTTAGAA TAGTATAGAT      3278

AGGCTACACT ACACTAAGTT GGACAGATAA AATAAGGGGT TGTAAACTTA GACTATTAAA      3338

AAAAGGGAGA GTGCTACT ATG ACA CGT CAA CAT CGA ACT TTT ACA CTC GAA       3389
                    Met Thr Arg Gln His Arg Thr Phe Thr Leu Glu
                     1               5                   10

TGT AAA CTG TAATTAGTGA AACTTTATGA CCACGGTAAA TATCGGGCGG                3438
Cys Lys Leu

ACACCGCTAA AGAATCATAA                                                   3458
```

(2) INFORMATION FOR SEQ ID NO:2:

    (i) SEQUENCE CHARACTERISTICS:
        (A) LENGTH: 448 amino acids
        (B) TYPE: amino acid
        (D) TOPOLOGY: linear

    (ii) MOLECULE TYPE: protein

    (xi) SEQUENCE DESCRIPTION: SEQ ID NO:2:

```
Met Cys Asp Ser Lys Asp Asn Ser Gly Val Ser Glu Lys Cys Gly Lys
 1               5                   10                  15

Lys Phe Thr Asn Tyr Pro Leu Asn Thr Thr Pro Thr Ser Leu Asn Tyr
                20              25                  30

Asn Leu Pro Glu Ile Ser Lys Lys Phe Tyr Asn Leu Lys Asn Lys Tyr
            35                  40                  45
```

Ser Arg Asn Gly Tyr Gly Leu Ser Lys Thr Glu Phe Pro Ser Ser Ile
    50                  55              60

Glu Asn Cys Pro Ser Asn Glu Tyr Ser Ile Met Tyr Asp Asn Lys Asp
    65              70              75              80

Pro Arg Phe Leu Ile Arg Phe Leu Leu Asp Asp Gly Arg Tyr Ile Ile
                85              90              95

Ala Asp Arg Asp Asp Gly Glu Val Phe Asp Glu Ala Pro Thr Tyr Leu
            100             105             110

Asp Asn Asn Asn His Pro Ile Ile Ser Arg His Tyr Thr Gly Glu Glu
        115             120             125

Arg Gln Lys Phe Glu Gln Val Gly Ser Gly Asp Tyr Ile Thr Gly Glu
    130             135             140

Gln Phe Phe Gln Phe Tyr Thr Gln Asn Lys Thr Arg Val Leu Ser Asn
145             150             155             160

Cys Arg Ala Leu Asp Ser Arg Thr Ile Leu Leu Ser Thr Ala Lys Ile
            165             170             175

Phe Pro Ile Tyr Pro Pro Ala Ser Glu Thr Gln Leu Thr Ala Phe Val
            180             185             190

Asn Ser Ser Phe Tyr Ala Ala Ala Ile Pro Gln Leu Pro Gln Thr Ser
        195             200             205

Leu Leu Glu Asn Ile Pro Glu Pro Thr Ser Leu Asp Asp Ser Gly Val
    210             215             220

Leu Pro Lys Asp Ala Val Arg Ala Val Lys Gly Ser Ala Leu Leu Pro
225             230             235             240

Cys Ile Ile Val His Asp Pro Asn Leu Asn Asn Ser Asp Lys Met Lys
            245             250             255

Phe Asn Thr Tyr Tyr Leu Leu Glu Tyr Lys Glu Tyr Trp His Gln Leu
            260             265             270

Trp Ser Gln Ile Ile Pro Ala His Gln Thr Val Lys Ile Gln Glu Arg
        275             280             285

Thr Gly Ile Ser Glu Val Val Gln Asn Ser Met Ile Glu Asp Leu Asn
    290             295             300

Met Tyr Ile Gly Ala Asp Phe Gly Met Tyr Phe Tyr Leu Arg Ser Ser
305             310             315             320

.

Gly Phe Lys Glu Gln Ile Thr Arg Gly Leu Asn Arg Pro Leu Ser Gln
                325                     330                 335

Thr Thr Thr Gln Leu Gly Glu Arg Val Glu Glu Met Glu Tyr Tyr Asn
            340                 345                 350

Ser Asn Asp Leu Asp Val Arg Tyr Val Lys Tyr Ala Leu Ala Arg Glu
        355                 360                 365

Phe Thr Leu Lys Arg Val Asn Gly Glu Ile Val Lys Asn Trp Val Ala
    370                 375                 380

Val Asp Tyr Arg Met Ala Gly Ile Gln Ser Tyr Pro Asn Ala Pro Ile
385             390                 395                 400

Thr Asn Pro Leu Thr Leu Thr Lys His Thr Ile Ile Arg Cys Glu Asn
            405                 410                 415

Ser Tyr Asp Gly His Ile Phe Lys Thr Pro Leu Ile Phe Lys Asn Gly
        420                 425                 430

Glu Val Ile Val Lys Thr Asn Glu Glu Leu Ile Pro Lys Ile Asn Gln
    435                 440                 445

(3) INFORMATION FOR SEQ ID NO:3:

        (i) SEQUENCE CHARACTERISTICS:
            (A) LENGTH: 370 amino acids
            (B) TYPE: amino acid
            (D) TOPOLOGY: linear

    (ii) MOLECULE TYPE: protein

    (xi) SEQUENCE DESCRIPTION: SEQ ID NO:3:

Met Arg Asn Leu Asp Phe Ile Asp Ser Phe Ile Pro Thr Glu Gly Lys
1               5                   10                  15

Tyr Ile Arg Val Met Asp Phe Tyr Asn Ser Glu Tyr Pro Phe Cys Ile
            20                  25                  30

His Ala Pro Ser Ala Pro Asn Gly Asp Ile Met Thr Glu Ile Cys Ser
        35                  40                  45

Arg Glu Asn Asn Gln Tyr Phe Ile Phe Phe Pro Thr Asp Asp Gly Arg
        50                  55                  60

Val Ile Ile Ala Asn Arg His Asn Gly Ser Val Phe Thr Gly Glu Ala
    65                  70                  75                  80

Thr Ser Val Val Ser Asp Ile Tyr Thr Gly Ser Pro Leu Gln Phe Phe
                85                      90            95

Arg Glu Phe Lys Arg Thr Met Ser Thr Tyr Tyr Leu Ala Ile Gln Asn
                100               105            110

Pro Glu Ser Ala Thr Asp Val Arg Ala Leu Glu Pro Asn Ser His Glu
            115               120            125

Leu Pro Ser Arg Leu Tyr Phe Thr Asn Asn Ile Glu Asn Asn Ser Asn
            130               135            140

Ile Leu Ile Ser Asn Lys Glu Gln Ile Tyr Leu Thr Leu Pro Ser Leu
145               150               155               160

Pro Glu Asn Glu Gln Tyr Pro Lys Thr Pro Val Leu Ser Gly Ile Asp
                165               170            175

Asp Ile Gly Pro Asn Gln Ser Glu Lys Ser Ile Ile Gly Ser Thr Leu
            180               185            190

Ile Pro Cys Ile Met Val Ser Asp Phe Ile Ser Leu Gly Glu Arg Met
            195               200            205

Lys Thr Thr Pro Tyr Tyr Tyr Val Lys His Thr Gln Tyr Trp Gln Ser
    210               215               220

Met Trp Ser Ala Leu Phe Pro Pro Gly Ser Lys Glu Thr Lys Thr Glu
225               230               235               240

Lys Ser Gly Ile Thr Asp Thr Ser Gln Ile Ser Met Thr Asp Gly Ile
                245               250            255

Asn Val Ser Ile Gly Ala Asp Phe Gly Leu Lys Phe Gly Asn Lys Thr
                260               265            270

Phe Gly Ile Lys Gly Gly Phe Thr Tyr Asp Thr Lys Thr Gln Ile Thr
            275               280            285

Asn Thr Ser Gln Leu Leu Ile Glu Thr Thr Tyr Thr Arg Glu Tyr Thr
    290               295               300

Asn Thr Glu Asn Phe Pro Val Arg Tyr Thr Gly Tyr Val Leu Ala Ser
305               310               315               320

Glu Phe Thr Leu His Arg Ser Asp Gly Thr Gln Val Asn Thr Ile Pro
                325               330            335

Trp Val Ala Leu Asn Asp Asn Tyr Thr Thr Ile Ala Arg Tyr Pro His
            340               345            350

Phe Ala Ser Glu Pro Leu Leu Gly Asn Thr Lys Ile Ile Thr Asp Asp
        355               360               365

Gln Asn
370

(4) INFORMATION FOR SEQ ID NO:4:

    (i) SEQUENCE CHARACTERISTICS:
        (A) LENGTH: 14 amino acids
        (B) TYPE: amino acid
        (D) TOPOLOGY: linear

    (ii) MOLECULE TYPE: protein

    (xi) SEQUENCE DESCRIPTION: SEQ ID NO:4:

Met Thr Arg Gln His Arg Thr Phe Thr Leu Glu Cys Lys Leu
  1          5            10


(5) INFORMATION FOR SEQ ID NO:5:

    (i) SEQUENCE CHARACTERISTICS:
        (A) LENGTH: 4133 base pairs
        (B) TYPE: nucleic acid
        (C) STRANDEDNESS: double
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:
        (A) ORGANISM: Bacillus sphaericus
        (B) STRAIN: SSII-1

    (ix) FEATURE:
        (A) NAME/KEY: CDS
        (B) LOCATION: 1..1020

    (ix) FEATURE:
        (A) NAME/KEY: CDS
        (B) LOCATION: 1207..3816

    (ix) FEATURE:
        (A) NAME/KEY: RBS
        (B) LOCATION: 1188..1198

(xi) SEQUENCE DESCRIPTION: SEQ ID NO:5:

```
GAT CAT GCT TAT TTG GAA AAA ATG CTG AGT TCG TTG CGT GAT AGT TTT        48
Asp His Ala Tyr Leu Glu Lys Met Leu Ser Ser Leu Arg Asp Ser Phe
 1               5                  10                  15

GTT CAA CCT TTC ACT ATT CAT GGC CAA ATC ACT TTT GTT CAA ATG AGT        96
Val Gln Pro Phe Thr Ile His Gly Gln Ile Thr Phe Val Gln Met Ser
               20                  25                  30

TTG GGA ATC GCA AGT TAT CCA ATT GAT GGA GTG ACT CAA GAA GAG CTT       144
Leu Gly Ile Ala Ser Tyr Pro Ile Asp Gly Val Thr Gln Glu Glu Leu
           35                  40                  45

CTC AAA CAT GCT GAT ATC GCC ATG TAT AAA GCG AAA GAA TTA GGG GGG       192
Leu Lys His Ala Asp Ile Ala Met Tyr Lys Ala Lys Glu Leu Gly Gly
       50                  55                  60

AAT AAC CAT CGT TTT TTT GAC GAA AAG ATG AAT GAA CTC GTT ATC AAA       240
Asn Asn His Arg Phe Phe Asp Glu Lys Met Asn Glu Leu Val Ile Lys
 65                  70                  75                  80

AAG GAC CAA ATA GAG CGA ATG ATA CGT CTA GCG CTA GAA AGA AAT GAA       288
Lys Asp Gln Ile Glu Arg Met Ile Arg Leu Ala Leu Glu Arg Asn Glu
                   85                  90                  95

TTT AGC GTC CAT TAT CAA CTG CAG ATA GAA GCC ACT ACT GGA AAG ATC       336
Phe Ser Val His Tyr Gln Leu Gln Ile Glu Ala Thr Thr Gly Lys Ile
               100                 105                 110

CGA GGG TTT GAG GCG TTA GTG CGC TGG AAA AGC CCT GAG CTT GGA CTT       384
Arg Gly Phe Glu Ala Leu Val Arg Trp Lys Ser Pro Glu Leu Gly Leu
           115                 120                 125

GTT TCT CCA GAA GAT TTT ATT CCG ATA GCA GAA AAA ACA GGC TTG ATT       432
Val Ser Pro Glu Asp Phe Ile Pro Ile Ala Glu Lys Thr Gly Leu Ile
       130                 135                 140

ACA CAA ATT GAC GAA TGG GTC ATG TAT CAA GCA TGC TTG AAA AAT GTC       480
Thr Gln Ile Asp Glu Trp Val Met Tyr Gln Ala Cys Leu Lys Asn Val
145                 150                 155                 160

GAA TTA CAA CAT CAA TTC GGT TAT CCC TTC CTG ATG TCG GTT AAT ATT       528
Glu Leu Gln His Gln Phe Gly Tyr Pro Phe Leu Met Ser Val Asn Ile
               165                 170                 175

TCG GCT TTA CAA TTA GGA AGA GCA GAT TTT GTG GAT AAA GTC CAG CTT       576
Ser Ala Leu Gln Leu Gly Arg Ala Asp Phe Val Asp Lys Val Gln Leu
           180                 185                 190
```

```
ATT TTG AAT GAA ACT AAG ATG AAG CCG GAA CAT TTA GAA ATT GAG ATT          624
Ile Leu Asn Glu Thr Lys Met Lys Pro Glu His Leu Glu Ile Glu Ile
        195             200             205

ACT GAG AGT ATC TTA GTT GAG TCA TTT GAA AGC TCT ATA TGC ATA CTG          672
Thr Glu Ser Ile Leu Val Glu Ser Phe Glu Ser Ser Ile Cys Ile Leu
        210             215             220

CGA AAA TTA AAG AAC CTA GGG GTG AAA ATT GCC CAG GAT GAT TTT GGT          720
Arg Lys Leu Lys Asn Leu Gly Val Lys Ile Ala Gln Asp Asp Phe Gly
225             230             235             240

ACC GGT TAT TCT TCA CTT AAC TAC TTA ACA TTA TTG CCG ATT CAT ACC          768
Thr Gly Tyr Ser Ser Leu Asn Tyr Leu Thr Leu Leu Pro Ile His Thr
                245             250             255

CTT AAA ATT GAT AGA TCC CTT ATT CAA AAT ATG ACA TCG GCA ACA GCA          816
Leu Lys Ile Asp Arg Ser Leu Ile Gln Asn Met Thr Ser Ala Thr Ala
            260             265             270

GAA AAA ACG ATA ATC GAA TCG ATT ATC CAC CTT GCC CAT AAA TTA GGT          864
Glu Lys Thr Ile Ile Glu Ser Ile Ile His Leu Ala His Lys Leu Gly
        275             280             285

CAT GAT GTA GTT GCA GAA GGA GTG GAG ACA AAA GAA CAG TAT ATC TTG          912
His Asp Val Val Ala Glu Gly Val Glu Thr Lys Glu Gln Tyr Ile Leu
        290             295             300

CTC AAA GAA TGG AAC TGC GAT TTT GTC CAG GGA TAC TAT TTC AGT AGG          960
Leu Lys Glu Trp Asn Cys Asp Phe Val Gln Gly Tyr Tyr Phe Ser Arg
305             310             315             320

CCT GTT TCG TCA GAC ATA CTT GTC GAA TGT CTG ACA GGG GAA AAG AAC         1008
Pro Val Ser Ser Asp Ile Leu Val Glu Cys Leu Thr Gly Glu Lys Asn
                325             330             335

TCC ACA AAT CAG TAAAAAATAA AGCTCTGTAA TTACAAAAAC AAACAACCAT            1060
Ser Thr Asn Gln
            340

GCCAATTTAG ATTATAAATT ATTGTTAGAA ATTCACACTA TCATCCCTTT CAAAATAAAT      1120

ATATAACTAT TATTTTTATT TAATGTTATT AAATACATAA TTTAATAACA TTAAATAAAT      1180

AAATACCAAA AAGAGGTGCA ATTGAT ATG GCT ATA AAA AAA GTA TTA AAA ATA       1233
                              Met Ala Ile Lys Lys Val Leu Lys Ile
                               1               5

ATT TTA GCA ATA ATT ATT ATA ATT AGT TGC CAA CTA CCA CTG AAT CAA        1281
Ile Leu Ala Ile Ile Ile Ile Ile Ser Cys Gln Leu Pro Leu Asn Gln
 10              15              20              25
```

22

```
AAA ACT GTT TAT GCT TCA CCT AAT TCT CCA AAA GAT AAC ACT TGG ATC        1329
Lys Thr Val Tyr Ala Ser Pro Asn Ser Pro Lys Asp Asn Thr Trp Ile
                30                  35                  40

CAA GCT GCT TCA CTT ACA TGG TTA ATG GAT ATG TCG AGT TTA TTG TAT        1377
Gln Ala Ala Ser Leu Thr Trp Leu Met Asp Met Ser Ser Leu Leu Tyr
                45                  50                  55

CAA CTG ATT TCA ACA AGA ATT CCC TCT TTT GCT TCT CCA AAT GGA TTA        1425
Gln Leu Ile Ser Thr Arg Ile Pro Ser Phe Ala Ser Pro Asn Gly Leu
                60                  65                  70

CAT ATG AGA GAA CAA ACT ATT GAT AGC AAT ACT GGA CAA ATA CAA ATA        1473
His Met Arg Glu Gln Thr Ile Asp Ser Asn Thr Gly Gln Ile Gln Ile
        75                  80                  85

GAT AAT GAG CAT AGG CTA TTA AGA TGG GAT AGG CGA CCG CCT AAT GAT        1521
Asp Asn Glu His Arg Leu Leu Arg Trp Asp Arg Arg Pro Pro Asn Asp
    90                  95                  100                 105

ATT TTT TTA AAT GGA TTT ATA CCT AGA GTA ACA AAC CAA AAT CTA TCT        1569
Ile Phe Leu Asn Gly Phe Ile Pro Arg Val Thr Asn Gln Asn Leu Ser
                110                 115                 120

CCA GTA GAA GAC ACA CAT CTT CTA AAT TAT TTA AGA ACA AAT TCT CCA        1617
Pro Val Glu Asp Thr His Leu Leu Asn Tyr Leu Arg Thr Asn Ser Pro
                125                 130                 135

TCC ATT TTT GTT TCC ACA ACT AGA GCT AGA TAC AAT AAT TTA GGT TTA        1665
Ser Ile Phe Val Ser Thr Thr Arg Ala Arg Tyr Asn Asn Leu Gly Leu
                140                 145                 150

GAA ATA ACA CCT TGG ACA CCT CAT AGT GCT AAT AAC AAT ATA ATT TAC        1713
Glu Ile Thr Pro Trp Thr Pro His Ser Ala Asn Asn Asn Ile Ile Tyr
        155                 160                 165

AGA TAT GAA ATT TTT GCT CCA GGT GGT ATT GAT ATT AAT GCA AGT TTT        1761
Arg Tyr Glu Ile Phe Ala Pro Gly Gly Ile Asp Ile Asn Ala Ser Phe
170                 175                 180                 185

TCA AGA AAC CAC AAT CCC TTT CCT AAC GAG GAT GAA ATA ACT TTT CCA        1809
Ser Arg Asn His Asn Pro Phe Pro Asn Glu Asp Glu Ile Thr Phe Pro
                190                 195                 200

GGG GGA ATT CGT CCT GAG TTT ATA CGT TCA ACA TAT GAA TAT CAC AAT        1857
Gly Gly Ile Arg Pro Glu Phe Ile Arg Ser Thr Tyr Glu Tyr His Asn
                205                 210                 215

GGT GAA ATT GTT AGA ATA TGG ATT AAC CCT AAT TTT ATT AAT CCA TCA        1905
Gly Glu Ile Val Arg Ile Trp Ile Asn Pro Asn Phe Ile Asn Pro Ser
        220                 225                 230
```

```
ACT CTA AAT GAT GTA TCA GGA CCC TCT AAT ATA AGC AAA GTA TTT TGG        1953
Thr Leu Asn Asp Val Ser Gly Pro Ser Asn Ile Ser Lys Val Phe Trp
        235             240             245

CAT GAA AAT CAT TCT GAA GGA AAT AAT ATG GAT TCT AAA GGT TTT ATA        2001
His Glu Asn His Ser Glu Gly Asn Asn Met Asp Ser Lys Gly Phe Ile
250             255             260             265

CTA GAT TTA GAT TAT AAT CAA GAT TTT GAC ATG TTT GCC CCC AAT GGA        2049
Leu Asp Leu Asp Tyr Asn Gln Asp Phe Asp Met Phe Ala Pro Asn Gly
            270             275             280

GAA ATA CCT AAT AAT AAT TTA TTA AAT AAT AAT AGT TTA AAT GTT ATA        2097
Glu Ile Pro Asn Asn Asn Leu Leu Asn Asn Asn Ser Leu Asn Val Ile
            285             290             295

CAA AAC TCA GAA TAT CAA ATC AAA AAT AAG AAG GAT AGG AAT ATT GTT        2145
Gln Asn Ser Glu Tyr Gln Ile Lys Asn Lys Lys Asp Arg Asn Ile Val
            300             305             310

GTA ACG TTA GAT TCT GAT TAT GGA GGA AGT CCA GTA GAG TCG TAT AAG        2193
Val Thr Leu Asp Ser Asp Tyr Gly Gly Ser Pro Val Glu Ser Tyr Lys
        315             320             325

AAT TTT GGT TTT GAA AAT CAA AAA TGG AAC ATC AAA TAC GAT AGC AAA        2241
Asn Phe Gly Phe Glu Asn Gln Lys Trp Asn Ile Lys Tyr Asp Ser Lys
330             335             340             345

AAA AAT GCT TAT AAA ATC TAC AAT AGA GAA ACC CCT ACT TTA CTA TTA        2289
Lys Asn Ala Tyr Lys Ile Tyr Asn Arg Glu Thr Pro Thr Leu Leu Leu
            350             355             360

AGT TGG AAT AGT AAC TCG TCT AAT GGT GAA CAA GTG ATT AGA GGT TAT        2337
Ser Trp Asn Ser Asn Ser Ser Asn Gly Glu Gln Val Ile Arg Gly Tyr
            365             370             375

ACT GAA AGT GGT TCA AAT AAT CAA TAT TGG ACA ATA GAA AAA AAT GTA        2385
Thr Glu Ser Gly Ser Asn Asn Gln Tyr Trp Thr Ile Glu Lys Asn Val
        380             385             390

AAT GGT TTT TAT AAA TTT AGA AAT CTT TCT GAC CCT AGC AAA ATA TTA        2433
Asn Gly Phe Tyr Lys Phe Arg Asn Leu Ser Asp Pro Ser Lys Ile Leu
        395             400             405

GAC TTA AAA GAT GGT AAC ACT CTA AAT AAA ACT CCT TTA GTC GTT TCA        2481
Asp Leu Lys Asp Gly Asn Thr Leu Asn Lys Thr Pro Leu Val Val Ser
410             415             420             425

AGT GAG AAC AGT AGC TCA TCA CAA GAG TGG TTA ATT GAA AAA ACT AAT        2529
Ser Glu Asn Ser Ser Ser Ser Gln Glu Trp Leu Ile Glu Lys Thr Asn
            430             435             440
```

```
TAT CAA ACT GTT AAA GAT GGT ACT TAT CAA GTA TCT TCA AAA TTA AAT        2577
Tyr Gln Thr Val Lys Asp Gly Thr Tyr Gln Val Ser Ser Lys Leu Asn
            445                 450                 455

GAA AAT AAA GTT ATT GAA CAA ATT TCT ACT AAC AAA GTT CAT ATT TTC        2625
Glu Asn Lys Val Ile Glu Gln Ile Ser Thr Asn Lys Val His Ile Phe
            460                 465                 470

TCA AAC TCG GAT AAA GAA AAT CAA GTT TGG AAT CTT ATC TAC AAC CCT        2673
Ser Asn Ser Asp Lys Glu Asn Gln Val Trp Asn Leu Ile Tyr Asn Pro
            475                 480                 485

ATC CTC AAA GCT TAT AAA ATT AAA AGC TTA AAG TAT CCT AAC TAT TCA        2721
Ile Leu Lys Ala Tyr Lys Ile Lys Ser Leu Lys Tyr Pro Asn Tyr Ser
490                 495                 500                 505

TTG GCT TGG GAT AGT AAT AAT ACT AGA ACA ATT GTA GCA GCT ACA GGG        2769
Leu Ala Trp Asp Ser Asn Asn Thr Arg Thr Ile Val Ala Ala Thr Gly
            510                 515                 520

GAT TAT AAT GAT CAA TAT TGG TTA ATA GAA AGA AAC GAG GAT AAT ACT        2817
Asp Tyr Asn Asp Gln Tyr Trp Leu Ile Glu Arg Asn Glu Asp Asn Thr
            525                 530                 535

TAT ATA ATT AGA AAT TAT GAA AAT AGA AAA ATT GTT TTA GAT TTA TCA        2865
Tyr Ile Ile Arg Asn Tyr Glu Asn Arg Lys Ile Val Leu Asp Leu Ser
            540                 545                 550

AAT GGT AGT ACT ACT GAT GGC AAT GGA TTA TTA GGA TTT GAA TTT CAT        2913
Asn Gly Ser Thr Thr Asp Gly Asn Gly Leu Leu Gly Phe Glu Phe His
            555                 560                 565

GGG GGA ATA AAT CAA AGA TGG ATC ATT AAG CCT TTT TCA TTT AAT TCT        2961
Gly Gly Ile Asn Gln Arg Trp Ile Ile Lys Pro Phe Ser Phe Asn Ser
570                 575                 580                 585

ATT CAA GAT GGG ATT TAT CAA TTT ATG ACC GTA ATT AAT CAG GAT TTA        3009
Ile Gln Asp Gly Ile Tyr Gln Phe Met Thr Val Ile Asn Gln Asp Leu
            590                 595                 600

ATT GCT GAC TTG ACG ACT AAT AAT TAT ACA ATT GCT ACG AAA ACA AAT        3057
Ile Ala Asp Leu Thr Thr Asn Asn Tyr Thr Ile Ala Thr Lys Thr Asn
            605                 610                 615

AAC TAT TCT AGT AAT CAA AAA TGG ACA GTA ACT TAT AAT GAT AAA AAA        3105
Asn Tyr Ser Ser Asn Gln Lys Trp Thr Val Thr Tyr Asn Asp Lys Lys
            620                 625                 630

CGT GCC TAT AAA ATT AGG AAT TTA CAA CAT GCT CAT CTC AGT TTA GCA        3153
Arg Ala Tyr Lys Ile Arg Asn Leu Gln His Ala His Leu Ser Leu Ala
            635                 640                 645
```

```
TGG GAT AGC AAT CAC TCA GAT AAA ATT TTT GGC GCT ACC GGA GAT TAT        3201
Trp Asp Ser Asn His Ser Asp Lys Ile Phe Gly Ala Thr Gly Asp Tyr
650             655             660             665

GAT GAT CAA TAT TGG ATT CCT ATT TTG CAA ACA GAT GGA TCA TTT ATT        3249
Asp Asp Gln Tyr Trp Ile Pro Ile Leu Gln Thr Asp Gly Ser Phe Ile
                670             675             680

TTT AGG AAT TAT AAA AAT CCA AAT AAG ATA TTT GGA ACA AAT GGT CAA        3297
Phe Arg Asn Tyr Lys Asn Pro Asn Lys Ile Phe Gly Thr Asn Gly Gln
            685             690             695

CCC ATT AAT GAT ATT CCG TTA AAG GCT CAA GAT GTA ACT GGA CAA AAT        3345
Pro Ile Asn Asp Ile Pro Leu Lys Ala Gln Asp Val Thr Gly Gln Asn
            700             705             710

AAT CAA AAG TGG TAT TTA AGA CAT TTA AAT TCT TCC AAT AAT TTT ACT        3393
Asn Gln Lys Trp Tyr Leu Arg His Leu Asn Ser Ser Asn Asn Phe Thr
        715             720             725

GGA TAC TTT AAC ATA TCC AGT AAA AAG AAT TTT AAT AAA ATT ATT ACT        3441
Gly Tyr Phe Asn Ile Ser Ser Lys Lys Asn Phe Asn Lys Ile Ile Thr
730             735             740             745

ATG AAC TCA AAT AAA ACT CAA GCT GTT ATT TTT GAC AAT ATT GGA ATT        3489
Met Asn Ser Asn Lys Thr Gln Ala Val Ile Phe Asp Asn Ile Gly Ile
            750             755             760

AAC AAT CAG TCA TGG AAA CTG AAA TAT AAT GAC AAT AAA AAT GCA TAT        3537
Asn Asn Gln Ser Trp Lys Leu Lys Tyr Asn Asp Asn Lys Asn Ala Tyr
            765             770             775

CAG ATT CAC ATT TTA GAT AAC TTT TTA TAC TTT CAA GGC GGC CAT AAT        3585
Gln Ile His Ile Leu Asp Asn Phe Leu Tyr Phe Gln Gly Gly His Asn
            780             785             790

ATT GTT GCT ACC ATG CGA AAT GTT ACT AAT GAT GAT TTA AGA AGT TAT        3633
Ile Val Ala Thr Met Arg Asn Val Thr Asn Asp Asp Leu Arg Ser Tyr
        795             800             805

TGG TAT GTA GAA TAT AAC TTT AAT AAA GAT GGG TTT ATA ATA AGA AAC        3681
Trp Tyr Val Glu Tyr Asn Phe Asn Lys Asp Gly Phe Ile Ile Arg Asn
810             815             820             825

GCA TTT GAT ACA AGC TAC GTA CTT GAT GTA TTT CAA GGA AAT TTT GCT        3729
Ala Phe Asp Thr Ser Tyr Val Leu Asp Val Phe Gln Gly Asn Phe Ala
            830             835             840

AAC AAT ACA CCT ATC ATC ACT TAT CAA AAC TAT TTA AAC GAC AAC CAG        3777
Asn Asn Thr Pro Ile Ile Thr Tyr Gln Asn Tyr Leu Asn Asp Asn Gln
            845             850             855
```

TTA TGG AAT TTT ATA CCT TCA TTA GGT GTA GAA CCT AGA TAGTAATGAC                    3826
Leu Trp Asn Phe Ile Pro Ser Leu Gly Val Glu Pro Arg
        860                 865                 870

TGTTTTATGC ATAGAAGACA AGCAAAAAAG AGGTTAACAG AGATATAATG AAATTATAAT    3886

AACATCTAAA TTGTAAATAA AGGGTGATAC ATATCGTAAT ATGTATCACC CTTTTTACTT    3946

TTGTACAACA ATCACTACCG ATTGTAGGTT AAAATAAAGT TAGATTTTTT TCTGTGATCT    4006

AATCTCCAAT AAAAACCTTT CCCTTTTCCT CTCTTAAAAT GCAGGTATCG ATTTAGTAGC    4066

CTTCTTAAAT TTTTAAAAAA GATGAATTAT CAATTTGATT TACCGTTAAA ATTTATTTAT    4126

ACTGCAG                                                              4133


(6) INFORMATION FOR SEQ ID NO:6:

    (i) SEQUENCE CHARACTERISTICS:
        (A) LENGTH: 340 amino acids
        (B) TYPE: amino acid
        (D) TOPOLOGY: linear

    (ii) MOLECULE TYPE: protein

    (xi) SEQUENCE DESCRIPTION: SEQ ID NO:6:

Asp His Ala Tyr Leu Glu Lys Met Leu Ser Ser Leu Arg Asp Ser Phe
 1               5                  10                  15

Val Gln Pro Phe Thr Ile His Gly Gln Ile Thr Phe Val Gln Met Ser
            20                  25                  30

Leu Gly Ile Ala Ser Tyr Pro Ile Asp Gly Val Thr Gln Glu Glu Leu
            35                  40                  45

Leu Lys His Ala Asp Ile Ala Met Tyr Lys Ala Lys Glu Leu Gly Gly
            50                  55                  60

Asn Asn His Arg Phe Phe Asp Glu Lys Met Asn Glu Leu Val Ile Lys
 65                  70                  75                  80

Lys Asp Gln Ile Glu Arg Met Ile Arg Leu Ala Leu Glu Arg Asn Glu
                85                  90                  95

Phe Ser Val His Tyr Gln Leu Gln Ile Glu Ala Thr Thr Gly Lys Ile
            100                 105                 110

Arg Gly Phe Glu Ala Leu Val Arg Trp Lys Ser Pro Glu Leu Gly Leu
            115                 120                 125

Val Ser Pro Glu Asp Phe Ile Pro Ile Ala Glu Lys Thr Gly Leu Ile
130                 135                 140

Thr Gln Ile Asp Glu Trp Val Met Tyr Gln Ala Cys Leu Lys Asn Val
145                 150                 155                 160

Glu Leu Gln His Gln Phe Gly Tyr Pro Phe Leu Met Ser Val Asn Ile
                165                 170                 175

Ser Ala Leu Gln Leu Gly Arg Ala Asp Phe Val Asp Lys Val Gln Leu
                180                 185                 190

Ile Leu Asn Glu Thr Lys Met Lys Pro Glu His Leu Glu Ile Glu Ile
                195                 200                 205

Thr Glu Ser Ile Leu Val Glu Ser Phe Glu Ser Ser Ile Cys Ile Leu
210                 215                 220

Arg Lys Leu Lys Asn Leu Gly Val Lys Ile Ala Gln Asp Asp Phe Gly
225                 230                 235                 240

Thr Gly Tyr Ser Ser Leu Asn Tyr Leu Thr Leu Leu Pro Ile His Thr
                245                 250                 255

Leu Lys Ile Asp Arg Ser Leu Ile Gln Asn Met Thr Ser Ala Thr Ala
                260                 265                 270

Glu Lys Thr Ile Ile Glu Ser Ile Ile His Leu Ala His Lys Leu Gly
                275                 280                 285

His Asp Val Val Ala Glu Gly Val Glu Thr Lys Glu Gln Tyr Ile Leu
290                 295                 300

Leu Lys Glu Trp Asn Cys Asp Phe Val Gln Gly Tyr Tyr Phe Ser Arg
305                 310                 315                 320

Pro Val Ser Ser Asp Ile Leu Val Glu Cys Leu Thr Gly Glu Lys Asn
                325                 330                 335

Ser Thr Asn Gln
                340


(7) INFORMATION FOR SEQ ID NO:7:

    (i) SEQUENCE CHARACTERISTICS:
        (A) LENGTH: 870 amino acids
        (B) TYPE: amino acid
        (D) TOPOLOGY: linear

    (ii) MOLECULE TYPE: protein

(xi) SEQUENCE DESCRIPTION: SEQ ID NO:7:

Met Ala Ile Lys Lys Val Leu Lys Ile Ile Leu Ala Ile Ile Ile Ile
1                   5                   10                  15

Ile Ser Cys Gln Leu Pro Leu Asn Gln Lys Thr Val Tyr Ala Ser Pro
                20                  25                  30

Asn Ser Pro Lys Asp Asn Thr Trp Ile Gln Ala Ala Ser Leu Thr Trp
                35                  40                  45

Leu Met Asp Met Ser Ser Leu Leu Tyr Gln Leu Ile Ser Thr Arg Ile
            50                  55                  60

Pro Ser Phe Ala Ser Pro Asn Gly Leu His Met Arg Glu Gln Thr Ile
    65                  70                  75                  80

Asp Ser Asn Thr Gly Gln Ile Gln Ile Asp Asn Glu His Arg Leu Leu
                    85                  90                  95

Arg Trp Asp Arg Arg Pro Pro Asn Asp Ile Phe Leu Asn Gly Phe Ile
                100                 105                 110

Pro Arg Val Thr Asn Gln Asn Leu Ser Pro Val Glu Asp Thr His Leu
                115                 120                 125

Leu Asn Tyr Leu Arg Thr Asn Ser Pro Ser Ile Phe Val Ser Thr Thr
    130                 135                 140

Arg Ala Arg Tyr Asn Asn Leu Gly Leu Glu Ile Thr Pro Trp Thr Pro
145                 150                 155                 160

His Ser Ala Asn Asn Asn Ile Ile Tyr Arg Tyr Glu Ile Phe Ala Pro
                165                 170                 175

Gly Gly Ile Asp Ile Asn Ala Ser Phe Ser Arg Asn His Asn Pro Phe
                180                 185                 190

Pro Asn Glu Asp Glu Ile Thr Phe Pro Gly Gly Ile Arg Pro Glu Phe
            195                 200                 205

Ile Arg Ser Thr Tyr Glu Tyr His Asn Gly Glu Ile Val Arg Ile Trp
    210                 215                 220

Ile Asn Pro Asn Phe Ile Asn Pro Ser Thr Leu Asn Asp Val Ser Gly
225                 230                 235                 240

Pro Ser Asn Ile Ser Lys Val Phe Trp His Glu Asn His Ser Glu Gly
                245                 250                 255

Asn Asn Met Asp Ser Lys Gly Phe Ile Leu Asp Leu Asp Tyr Asn Gln
            260                 265                 270

Asp Phe Asp Met Phe Ala Pro Asn Gly Glu Ile Pro Asn Asn Asn Leu
275 280 285

Leu Asn Asn Asn Ser Leu Asn Val Ile Gln Asn Ser Glu Tyr Gln Ile
290 295 300

Lys Asn Lys Lys Asp Arg Asn Ile Val Val Thr Leu Asp Ser Asp Tyr
305 310 315 320

Gly Gly Ser Pro Val Glu Ser Tyr Lys Asn Phe Gly Phe Glu Asn Gln
325 330 335

Lys Trp Asn Ile Lys Tyr Asp Ser Lys Lys Asn Ala Tyr Lys Ile Tyr
340 345 350

Asn Arg Glu Thr Pro Thr Leu Leu Leu Ser Trp Asn Ser Asn Ser Ser
355 360 365

Asn Gly Glu Gln Val Ile Arg Gly Tyr Thr Glu Ser Gly Ser Asn Asn
370 375 380

Gln Tyr Trp Thr Ile Glu Lys Asn Val Asn Gly Phe Tyr Lys Phe Arg
385 390 395 400

Asn Leu Ser Asp Pro Ser Lys Ile Leu Asp Leu Lys Asp Gly Asn Thr
405 410 415

Leu Asn Lys Thr Pro Leu Val Val Ser Ser Glu Asn Ser Ser Ser Ser
420 425 430

Gln Glu Trp Leu Ile Glu Lys Thr Asn Tyr Gln Thr Val Lys Asp Gly
435 440 445

Thr Tyr Gln Val Ser Ser Lys Leu Asn Glu Asn Lys Val Ile Glu Gln
450 455 460

Ile Ser Thr Asn Lys Val His Ile Phe Ser Asn Ser Asp Lys Glu Asn
465 470 475 480

Gln Val Trp Asn Leu Ile Tyr Asn Pro Ile Leu Lys Ala Tyr Lys Ile
485 490 495

Lys Ser Leu Lys Tyr Pro Asn Tyr Ser Leu Ala Trp Asp Ser Asn Asn
500 505 510

Thr Arg Thr Ile Val Ala Ala Thr Gly Asp Tyr Asn Asp Gln Tyr Trp
515 520 525

Leu Ile Glu Arg Asn Glu Asp Asn Thr Tyr Ile Ile Arg Asn Tyr Glu
530 535 540

Asn Arg Lys Ile Val Leu Asp Leu Ser Asn Gly Ser Thr Thr Asp Gly
545                  550              555              560

Asn Gly Leu Leu Gly Phe Glu Phe His Gly Gly Ile Asn Gln Arg Trp
             565              570              575

Ile Ile Lys Pro Phe Ser Phe Asn Ser Ile Gln Asp Gly Ile Tyr Gln
         580              585              590

Phe Met Thr Val Ile Asn Gln Asp Leu Ile Ala Asp Leu Thr Thr Asn
         595              600              605

Asn Tyr Thr Ile Ala Thr Lys Thr Asn Asn Tyr Ser Ser Asn Gln Lys
     610              615              620

Trp Thr Val Thr Tyr Asn Asp Lys Lys Arg Ala Tyr Lys Ile Arg Asn
625              630              635              640

Leu Gln His Ala His Leu Ser Leu Ala Trp Asp Ser Asn His Ser Asp
             645              650              655

Lys Ile Phe Gly Ala Thr Gly Asp Tyr Asp Asp Gln Tyr Trp Ile Pro
         660              665              670

Ile Leu Gln Thr Asp Gly Ser Phe Ile Phe Arg Asn Tyr Lys Asn Pro
     675              680              685

Asn Lys Ile Phe Gly Thr Asn Gly Gln Pro Ile Asn Asp Ile Pro Leu
     690              695              700

Lys Ala Gln Asp Val Thr Gly Gln Asn Asn Gln Lys Trp Tyr Leu Arg
705              710              715              720

His Leu Asn Ser Ser Asn Asn Phe Thr Gly Tyr Phe Asn Ile Ser Ser
             725              730              735

Lys Lys Asn Phe Asn Lys Ile Ile Thr Met Asn Ser Asn Lys Thr Gln
         740              745              750

Ala Val Ile Phe Asp Asn Ile Gly Ile Asn Asn Gln Ser Trp Lys Leu
         755              760              765

Lys Tyr Asn Asp Asn Lys Asn Ala Tyr Gln Ile His Ile Leu Asp Asn
     770              775              780

Phe Leu Tyr Phe Gln Gly Gly His Asn Ile Val Ala Thr Met Arg Asn
785              790              795              800

Val Thr Asn Asp Asp Leu Arg Ser Tyr Trp Tyr Val Glu Tyr Asn Phe
             805              810              815

Asn Lys Asp Gly Phe Ile Ile Arg Asn Ala Phe Asp Thr Ser Tyr Val
      820                   825               830

Leu Asp Val Phe Gln Gly Asn Phe Ala Asn Asn Thr Pro Ile Ile Thr
      835               840               845

Tyr Gln Asn Tyr Leu Asn Asp Asn Gln Leu Trp Asn Phe Ile Pro Ser
    850               855               860

Leu Gly Val Glu Pro Arg
865                 870


(8) INFORMATION FOR SEQ ID NO:8:

    (i) SEQUENCE CHARACTERISTICS:
        (A) LENGTH: 27 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:
        (A) ORGANISM: Bacillus thuringiensis
        (B) STRAIN: var. israelensis


    (xi) SEQUENCE DESCRIPTION: SEQ ID NO:8:

GGCGAATTCA TGAACTCAGG CTATCCG                                        27


(9) INFORMATION FOR SEQ ID NO:9:

    (i) SEQUENCE CHARACTERISTICS:
        (A) LENGTH: 40 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: YES

    (vi) ORIGINAL SOURCE:
        (A) ORGANISM: Bacillus thuringiensis
        (B) STRAIN: var. israelensis

(xi) SEQUENCE DESCRIPTION: SEQ ID NO:9:

TAGAAAACAT AAAAAATAGA TATCTAGATC TCGAGGATCC                                    40